# EUROPEAN PATENT SPECIFICATION

(11) **EP 4 410 216 B1**
(45) Date of publication and mention of the grant of the patent: **19.11.2025**
(21) Application number: 24155498.9
(22) Date of filing: 02.02.2024
(51) Int. Cl.: A61B 8/06, A61B 5/026, A61B 5/1455, A61B 5/00, A61B 8/08, A61B 8/00, A61H 31/00

(54) **ENHANCING PATIENT MONITORING WITH MULTIPLE SENSORS**
VERBESSERUNG DER PATIENTENÜBERWACHUNG MIT MEHREREN SENSOREN
AMÉLIORATION DE LA SURVEILLANCE D'UN PATIENT AU MOYEN DE CAPTEURS MULTIPLES

(30) Priority: 03.02.2023 US 202363443301 P; 22.12.2023 US 202363614402 P
(43) Date of publication of application: 07.08.2024
(73) Proprietor: Stryker Corporation, Portage, MI 49002-9711 (US)
(72) Inventor: SIEDENBURG, Clinton T., Kalamazoo, 49002 (US); CHAPMAN, Fred W., Kalamazoo, 49002 (US); PIRAINO, Daniel W., Kalamazoo, 49002 (US); TAYLOR, Tyson G., Kalamazoo, 49002 (US); WALKER, Robert G., Kalamazoo, 49002 (US); MARX, Robert P., Kalamazoo, 49002 (US)
(74) Representative: V.O.

(56) References cited:
- WO-A1-2017/056042
- WO-A1-2017/211814
- US-A1- 2019 053 975
- US-A1- 2020 113 455
- US-A1- 2021 169 736
- US-A1- 2022 142 858

## Description

### CROSS-REFERENCE TO RELATED APPLICATIONS

This application claims priority to U.S. Provisional App. No. 63/443,301 (titled "Blood Flow Monitors" and filed on February 3, 2023) as well as U.S. Provisional App. No. 63/614,402 (titled "Enhancing Patient Monitoring With Multiple Sensors" and filed on December 22, 2023).

### BACKGROUND

Cardiopulmonary resuscitation (CPR) is an emergency medical procedure to restore blood circulation and oxygenation in a subject suffering from cardiac arrest. CPR includes repeatedly administering chest compressions to pump blood through the body. Chest compressions can be delivered manually or by mechanical chest compression devices (MCCDs). CPR is often administered by lay bystanders at the scene of a medical emergency. CPR is essential to maintaining blood flow to vital organs while the patient is transported to a clinical setting for further care. Monitoring a condition of the subject during CPR can provide useful information about the efficacy of CPR. Relevant prior art is disclosed in US2022/142858 and US2021/169736.

### SUMMARY

According to a first aspect of the present invention there is provided a medical device, comprising:
a first sensor configured to detect a first physiological parameter of a subject;
a second sensor configured to detect a second physiological parameter of the subject,;an output device; and
a processor configured to:
   determine, by analyzing the first physiological parameter, that the subject is receiving a treatment during a first time interval;
   in response to determining that the subject is receiving the treatment during the first time interval, determine, by analyzing the second physiological parameter detected during a second time interval that is different than the first time interval, a condition of the subject; and
   cause the output device to output an indication of the condition;
   wherein the second physiological parameter includes a blood flow rate, a net flow volume, or a volumetric flow rate,wherein the medical device further includes a third sensor configured to detect a blood pressure of the subject, and
   the processor is configured to analyze the second physiological parameter and
   the blood pressure of the subject to determine the condition of the subject.

Optionally, the treatment comprises a chest compression, a pace pulse, assisted ventilation, or an electrical shock. Optionally, the condition comprises a pulse, pulseless electrical activity (PEA), a seizure, a spontaneous breath, or an arrhythmia.

Optionally, the processor is configured to determine, by analyzing the second physiological parameter detected during the second time interval that is different than the first time interval, the condition of the subject by identifying, in the second physiological parameter, an artifact indicative of the condition.

Optionally, the output device is configured to output the indication of the condition by transmitting, to a mechanical chest compression device, an instruction to cease applying chest compressions to the subject.

Optionally, the output device is configured to display a waveform indicative of the second physiological parameter, the waveform comprising a first portion corresponding to the first time interval and a second portion corresponding to the second time interval. Optionally, the first portion comprises a different color or style than the second portion.

Optionally, the first sensor comprises an accelerometer configured to detect an acceleration of a chest of a subject over the first time interval and the second time interval. Optionally, the second sensor comprises a pulse sensor configured to detect a parameter indicative of blood flow through a blood vessel of the subject over the first time interval and the second time interval. The processor can be configured to detect, by analyzing the acceleration of the chest of the subject, a first chest compression performed on the subject during the first time interval. The processor can be configured to detect, by analyzing the acceleration of the chest of the subject, an absence of a second chest compression performed on the subject during the second time interval. The processor can be configured to, in response to detecting the absence of the second chest compression performed on the subject during the second time interval, detect, by analyzing the parameter indicative of blood flow through the blood vessel of the subject, a pulse of the subject during the second time interval. The processor can be configured to cause the output device to output a signal indicating the pulse.

Optionally, the processor is configured to detect the pulse of the subject during the second time interval by calculating a derivative of the blood flow during the second time interval with respect to time, and determining that the derivative decreases and crosses zero.

Optionally, the pulse sensor comprises an emitter configured to output an incident beam, and a receiver configured to detect the parameter indicative of blood flow through the blood vessel of the subject by detecting a reflection or scatter of the incident beam from the blood flow through the blood vessel of the subject. The incident beam can comprises ultrasound and/or infrared light.

According to a further aspect of the present invention there is provided a method, comprising:
identifying a first physiological parameter of a subject detected by a first sensor;
identifying a second physiological parameter of the subject detected by a second sensor,
wherein the second physiological parameter includes a blood flow rate, a net flow volume, or a volumetric flow rate;
detecting, by a third sensor, a blood pressure of the subject;
determining, by analyzing the first physiological parameter, that the subject is receiving a treatment during a first time interval;
in response to determining that the subject is receiving the treatment during the first time interval,
determining, by analyzing the second physiological parameter detected during a second time interval that is different than the first time interval, a condition of the subject; and
outputting an indication of the condition,
wherein the condition of the subject is determined by analyzing the second physiological parameter and the blood pressure of the subject.

Optionally, the second physiological parameter comprises a volume of a blood vessel of the subject. Determining the second physiological parameter of the subject detected by a second sensor can comprise determining the second physiological parameter during a first instance of a cyclical event and during a second instance of the cyclical event. The method can further comprise: detecting a blood pressure of the blood vessel of the subject during the first instance of a cyclical event and during the second instance of the cyclical event, the first instance and the second instance occurring during the second time interval; determining a slope of a first pressure-volume (PV) loop corresponding to the blood pressure and the second physiological parameter during the first instance of the cyclical event; determining a slope of a second PV loop corresponding to the blood pressure and the second physiological parameter during the second instance of the cyclical event; and determining a difference between the slope of the first PV loop and the slope of the second PV loop, wherein determining the condition of the subject comprises determining that the difference between the slope of the first PV loop and the slope of the second PV loop is less than a first threshold or greater than a second threshold, and wherein the cyclical event comprises a cardiac cycle, a chest compression cycle, or a cardiopulmonary resuscitation (CPR) cycle.

Optionally, the treatment comprises a chest compression, a pace pulse, assisted ventilation, or an electrical shock. The condition can comprise a pulse, pulseless electrical activity (PEA), a seizure, a spontaneous breath, administration of a medication, or an arrhythmia.

Optionally, determining, by analyzing the second physiological parameter detected during the second time interval that is different than the first time interval, the condition of the subject comprises: detecting, in the second physiological parameter, an artifact indicative of the condition.

Optionally, outputting the indication of the condition comprises transmitting, to a mechanical chest compression device, an instruction to cease applying chest compressions to the subject.

Optionally, the treatment being a first treatment, outputting the indication of the condition comprises outputting, to a user, an instruction to administer a second treatment to the subject.

Optionally, the method further comprises displaying a waveform indicative of the second physiological parameter, the waveform comprising a first portion corresponding to the first time interval and a second portion corresponding to the second time interval, wherein the first portion comprises a different color or style than the second portion.

Optionally, outputting the indication of the condition comprises outputting the indication that the condition is stable, worsening, or improving.

It will be appreciated that any of the aspects, features and options described herein can be combined. It will particularly be appreciated that any of the aspects, features and options described in view of the medical device apply equally to the monitoring system and method, and vice versa.

### BRIEF DESCRIPTION OF THE DRAWINGS

FIG. 1 illustrates an example environment in which a mechanical chest compression device is administering chest compressions to a subject experiencing cardiac arrest.
FIG. 2 illustrates an example method for determining a condition of a subject.
FIG. 3 illustrates an example of an environment in which a condition of a subject is evaluated based on a blood pressure and a blood flow parameter of the subject.
FIG. 4 illustrates an example environment for monitoring blood flow through one or more blood vessels of a subject.
FIG. 5 illustrates various placements of the flow monitor on the body of a patient.
FIG. 6 illustrates the structure of an example flow monitor.
FIG. 7 illustrates an example of an external defibrillator configured to perform various functions described herein.
FIG. 8 illustrates a chest compression device configured to perform various functions described herein.
FIG. 9 illustrates a medical device configured to perform various functions described herein.

### DETAILED DESCRIPTION

Implementations of the present disclosure are directed to specific improvements in the technical field of patient monitoring and emergency medicine. In particular, implementations of the present disclosure relate to monitoring a condition of a patient while they are undergoing an emergency medical procedure.

Various implementations described herein relate to systems, devices, and methods for processing and analyzing a physiological parameter containing a treatment artifact based on identifying a treatment administered. Various methods described herein filter or temporally gate the physiological parameter based on determining a parameter of the treatment. Accordingly, the physiological parameter can be determined more accurately. Accurate measurement of the physiological parameter can provide insight into the effectiveness of the treatment and inform decisions to modify treatment (e.g., adjust a treatment parameter). The use of various implementations described herein can improve accuracy of patient monitoring and improve the efficacy of medical treatments.

As used herein, the terms "blood flow," "blood flow parameters," and their equivalents, may refer to one or more physiological parameters indicative of a movement of blood through a blood vessel. For example, the term "blood velocity" may refer a change in position with respect to time (i.e., distance per time, such as meters per second) of one or more blood cells moving in a blood vessel. The term "blood speed" may refer to a magnitude of a velocity (i.e., distance per time) of one or more blood cells moving in a blood vessel. The term "velocity profile," for example, may refer to a velocity of blood in a blood vessel with respect to a location within the blood vessel, such as a velocity of blood in a blood vessel with respect to a location along a cross-section of the blood vessel. The term "flow rate," and its equivalents, may refer to a volume or mass of blood that passes a boundary (e.g., a cross-section of a blood vessel) with respect to time. The terms "net flow," "net flow volume," and their equivalents, may refer to a volume or mass of blood that passes a boundary during a discrete time interval, such as during a cardiac cycle. A net flow volume can be calculated by integrating a flow rate over the time interval. Unless otherwise specified explicitly or by context, the term "blood flow" may refer to blood velocity, blood speed, velocity profile, flow rate, net flow, or any other flow-related parameter described herein.

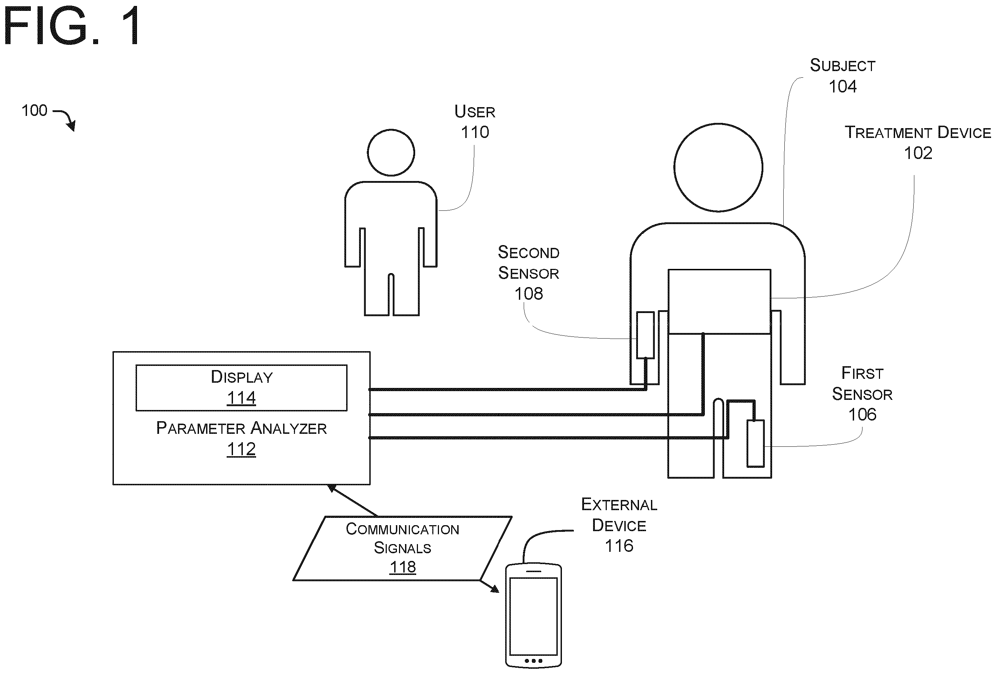

FIG. 1 illustrates an example environment 100 in which a treatment device 102 is administering an emergency medical procedure to a subject 104 experiencing cardiac arrest. A first sensor 106 and second sensor 108 are disposed on the subject 104 to monitor a condition of the subject 104. During cardiac arrest, the heart of the subject 104 stops effectively pumping blood to a body of the subject 104. For instance, the subject 104 may have an arrhythmia (e.g., ventricular fibrillation (VF), ventricular tachycardia (VT), or the like) that prevents the heart from effectively pumping blood. The treatment device 102 may be configured to administer a treatment (e.g., the emergency medical procedure) to restore blood circulation in the subject 104 until the subject 104 can receive further treatment in a clinical environment, such as a hospital, or until the subject is successfully resuscitated.

In some examples, the treatment device 102 is configured to deliver a treatment to the subject 104. The treatment, for instance, includes chest compressions, pacing pulses, assisted ventilation, an electrical shock, or any combination thereof. The treatment device 102 may include a mechanical chest compression device, a defibrillator, a ventilator, a pocket mask, or a bag-valve-mask resuscitator. In some cases, the treatment device 102 is a portable medical device configured to be operated outside of a clinical environment by a user 110. For example, the environment could be at the scene of a car crash, in an airport terminal, in a residence, or some other place in which the subject 104 is experiencing a sudden medical emergency. The user 110 may bring the treatment device 102 to the subject 104 in response to the subject 104 losing consciousness. The treatment device 102, in some instances, administers the treatment to the subject 104 before or while the subject 104 is transferred to a clinical environment for further treatment.

According to some implementations, the first sensor 106 is configured to detect a physiological parameter of the subject 104. The first sensor 106 may be disposed on or near the subject 104. According to various implementations, a condition of the subject 104 can be determined based on the physiological parameter. In some examples, the first sensor 106 is configured to determine the condition of the subject 104. As used herein, the term "physiological parameter," and its equivalents, may refer to a metric indicative of a physical condition of an individual, such as a patient or other type of subject. Examples of physiological parameters include an electrocardiogram (ECG), an impedance (e.g., a transthoracic impedance), a force administered to the patient, a blood pressure, an airway parameter (e.g., a capnograph, an end tidal gas parameter, a flow rate, etc.), a blood oxygenation (e.g., a pulse oximetry value, a regional oximetry value, a partial pressure of oxygen, a partial pressure of carbon dioxide, etc.), an electroencephalogram (EEG), a temperature, a heart sound, a blood flow rate, a physiological geometry (e.g., a shape of a blood vessel, an inner ear shape, etc.), a heart rate, a pulse rate, or another type of metric indicative of the condition of the subject 104. In some examples, the condition of the subject 104 includes a pathology, an arrhythmia, a pulse, pulseless electrical activity (PEA), a seizure, a spontaneous breath, or a return of spontaneous circulation (ROSC).

In some implementations, the first sensor 106 is configured to detect a blood flow of the subject 104. In some examples, the first sensor 106 may be configured to detect a blood flow to the brain of the subject 104. The first sensor 106 may include a transducer (e.g., an ultrasound transducer), an electromagnetic flowmeter, a pulse oximeter, an arterial doppler probe, a laser Doppler flowmetry device, or another sensor configured to detect the blood flow of the subject 104. In some implementations, the first sensor 106 is configured to detect an ECG of the subject 104. The first sensor 106 may include electrodes, an ECG monitor, an automated external defibrillator (AED), a monitor-defibrillator, or another device configured to detect an ECG. In some implementations, the first sensor 106 is configured to detect a blood oxygenation. The first sensor 106 may include a pulse oximeter or a blood gas analyzer. In some implementations, the first sensor 106 is configured to detect an airway parameter, such as a capnograph, an end tidal gas parameter, a flow rate, an inspiratory or expiratory pressure, or another airway parameter. The first sensor 106 may include a blood gas analyzer, a capnography device, a transcutaneous blood gas monitor, a manometer, a flow meter, or another sensor configured to detect an airway parameter. In some examples, the first sensor 106 is configured to detect a pulse. The first sensor 106 may include an emitter configured to output an incident beam (e.g., ultrasound and/or infrared light), and a receiver configured to detect a reflection or scatter of the incident beam.

In some cases, it may be advantageous to determine when the treatment (e.g., chest compressions) is being administered to the subject 104 in order to more accurately determine the condition of the subject 104. For example, during a time in which the treatment device 102 is administering chest compressions to the subject 104, the physiological parameter detected by the first sensor 106 may include a motion artifact associated with the chest compressions. In some examples, return of spontaneous circulation (ROSC) is difficult to identify when a pulse of the subject 104 is weak. ROSC can be detected using arterial pulse palpitation, ECG, capnography, end-tidal carbon dioxide monitoring, or blood pressure monitoring. In patients undergoing chest compressions, ROSC can be detected within chest compression pauses. In this case, it may be beneficial to identify the pulse when chest compressions are not occurring, and to detect whether ROSC is present during the chest compression pause. In various instances, it is useful to detect the physiological parameter while chest compressions are being administered. For example, end-tidal carbon dioxide (CO₂) in the airway of the subject 104 may be detected during chest compressions to determine the efficacy of the treatment. An increase in end-tidal CO₂ of the subject 104 indicates restoration of blood flow and effective chest compressions. If the end-tidal CO₂ level decrease or remain unchanged during chest compressions, a chest compression parameter (e.g., a position, frequency, depth, or duty cycle of chest compressions) should be adjusted to improve the efficacy of treatment.

These issues can be addressed, for example, by using the second sensor 108 to identify when the treatment (e.g., a chest compression) is administered to the subject 104. Based on identifying that chest compressions are being administered, measurement of a physiological parameter may be temporally gated to determine a condition of the subject 104 more accurately. For instance, an end-tidal carbon dioxide measurement may be temporally gated during a time window in which the treatment device 102 has paused chest compressions. By temporarily gating the partial pressure of carbon dioxide measurement, the condition of the subject 104 and the efficacy of the chest compressions may be assessed more accurately. In some examples, a compression artifact associated with the chest compressions can be identified and removed from the physiological parameter detected by the first sensor 106. Based on identifying the compression artifact, the physiological parameter can be filtered to remove the compression artifact. For example, one or more frequencies associated from the compression artifact can be removed by applying a filter to the physiological parameter. The filter, in some cases, is identified or designed based on the compression artifact. Removing the compression artifact, for instance, may enable a more accurate determination of the condition of the subject 104 in view of the physiological parameter. For example, an artifact associated with the frequency of chest compressions can be removed from an ECG detected by the first sensor 106.

According to various implementations, the second sensor 108 is configured to detect a parameter (also referred to as a "treatment parameter") associated with a treatment administered to the subject 104. In some examples, the treatment parameter is another physiological parameter of the subject 104. The second sensor 108 may include an accelerometer, a microphone, or a device configured to detect the treatment parameter of the subject 104. In various cases, the second sensor 108 is configured to detect an impedance, an energy level, or a voltage. In some examples, the second sensor 108 is disposed on or located near the subject 104.

According to some implementations, the first sensor 106 and the second sensor 108 are connected to a transceiver configured to transmit signals indicative of the treatment to a parameter analyzer 112. The parameter analyzer 112, in some cases, is included in a medical device (e.g., a monitor-defibrillator). The transceiver is configured to transmit and receive communication signals using one or more communication network(s). In some implementations, the transceiver is configured to transmit signals to the parameter analyzer 112 in a wired fashion and/or wirelessly. For example, the communication network(s) includes one or more wireless networks that include a 3GPP network, such as an LTE radio access network (RAN) (e.g., over one or more LTE bands), an NR RAN (e.g., over one or more NR bands), or a combination thereof. In some instances, the communication signals are electromagnetic (EM) signals, radio waves, or the like. In some implementations, the transceiver is configured to communicate with external devices by transmitting and/or receiving signals wirelessly. The external devices, for example, includes at least one of a sensor (e.g., the first sensor 106, the second sensor 108), a medical device (e.g., the treatment device 102), a computing device (e.g., the parameter analyzer 112), a mobile device, or a server. Examples of wireless networks include WI-FI^{®}, cellular networks, wireless local area networks (WLANs), and BLUETOOTH^{®}. In various examples, the transceiver includes a network interface card (NIC), a network adapter, a local area network (LAN) adapter, or a physical, virtual, or logical address to connect to the various external devices and/or systems. In various examples, the transceiver includes any sort of wireless transceivers capable of engaging in wireless communication (e.g., RF communication). In some examples, the transceiver transmits radio waves to the parameter analyzer 112 via a cell tower. In some cases, the transceiver is connected to a wireless modem, such as a modem for engaging in WI-FI^{®}, WIGIG^{®}, WIMAX^{®}, BLUETOOTH^{®}, or infrared communication. In some examples, the transceiver is configured to transmit signals in a wired fashion, such as by using a cable that connects the transceiver to the parameter analyzer 112. The transceiver and the parameter analyzer 112. in some implementations, include ports configured to receive connectors attached to the cable. In various examples, the transceiver includes a NIC to transmit data over the cable. Examples of wired connections include USB, USB-C, mini-USB, micro-USB, serial ports, and custom cables, among other examples.

Based on receiving signals indicative of the treatment, the parameter analyzer 112 is configured to determine the condition of the subject 104 in various examples. According to some implementations, the parameter analyzer 112 is connected to the first sensor 106 and the second sensor 108. The parameter analyzer 112 may be part of a medical device, such as the treatment device 102, the first sensor 106, the second sensor 108, or another medical device configured to monitor or treat the subject. In some implementations, the parameter analyzer 112 is part of a computing device (e.g., a mobile device, or the like). The parameter analyzer 112 may include one or more processors.

The first sensor 106 and the second sensor 108 are configured to transmit signals indicative of the physiological parameter and the treatment parameter, respectively, to the parameter analyzer 112. Based on receiving the signals indicative of the treatment, the parameter analyzer 112 is configured to remove an artifact associated with the treatment (also referred to as a "treatment artifact") from the physiological parameter. In some examples, the treatment includes chest compressions, and the treatment artifact is a frequency, depth, location, or time period of chest compressions. In some examples, the treatment includes an electrical shock or a pace pulse, and the treatment artifact is a frequency, energy level, or time period of treatment.

The parameter analyzer 112 is configured, in some examples, to filter and/or temporally gate the physiological parameter based on analyzing the physiological parameter and the treatment parameter. As used herein, the term "filter" and its equivalents refer to attenuating or eliminating specific components in a signal and/or data. In some cases, a filter can be used to remove one or more artifacts in the signal and/or data. Filtering can be performed in the time domain and/or the frequency domain. Filters may be analog and/or digital. Examples of filters include high-pass filters, low-pass filters, band-pass filters, band-stop filters (e.g., a notch filter), comb filters, Butterworth filters, Chebyshev filters, elliptic filters, Bessel filters, etc. For example, the second sensor 108 may include an accelerometer, and the parameter analyzer 112 may be configured to determine a treatment frequency (e.g., a chest compression frequency) from the accelerometer measurements. The parameter analyzer 112 may be configured to identify and remove an artifact caused by the treatment from data representative of the physiological parameter by applying a band reject filter centered around the chest compression frequency or by applying a comb filter that includes the chest compression frequency and one or more harmonics of the chest compression frequency. In some implementations, an artifact may be detected in a signal (i.e., a time domain representation of a signal). The artifact, in some examples, is a periodic artifact. Based on detecting the artifact, the artifact may be attenuated or eliminated from the signal, such as by subtracting the artifact from the signal. Other techniques known in the art of signal processing can be used to filter or gate the physiological parameter.

Based on removing the treatment artifact from the physiological parameter, the parameter analyzer 112 may determine the condition of the subject 104. In some cases, the parameter analyzer 112 may temporally gate the physiological parameter to determine the condition of the subject 104. As used herein, "temporally gate" and its equivalents refer to excluding signals outside of a particular time window. For example, the parameter analyzer 112 may temporally gate the physiological parameter during a time window in which the treatment device 102 has paused treatment or between treatments performed by the treatment device 102 to eliminate noise or artifacts associated with the treatment. The parameter analyzer 112, according to some implementations, outputs an indication of the condition on a display 114. In some examples, the parameter analyzer 112 may transmit signals indicative of the condition of the subject 104 to an external device 116 or to a medical device (e.g., the treatment device 102, the first sensor 106, the second sensor 108, or another medical device).

In some implementations, the parameter analyzer 112 is configured to determine, based on the treatment parameter, a first time period during which a treatment (e.g., chest compressions) is being administered and a second time period during which the treatment is not being administered. In some examples, the parameter analyzer 112 may be configured to analyze the physiological parameter during the first time period. For example, a blood flow parameter may be analyzed during the first time period to determine an efficacy of the treatment. In some examples, the parameter analyzer 112 may be configured to analyze the physiological parameter during the second time period. For example, a pulse of the subject 104 may be more accurately detected when chest compressions are paused, and thus the parameter analyzer 112 may be configured to analyze the blood flow of the subject 104 during the second time period. The parameter analyzer 112 may be configured to determine a volumetric flow rate, a velocity, a pressure, or another characteristic of blood flow. The parameter analyzer 112 may calculate a derivative of blood flow and determine a time interval at which the derivative crosses zero. Based on determining the time interval, the parameter analyzer 112 may determine the pulse of the subject 104.

According to the invention, the parameter analyzer 112 is configured to analyze a blood pressure of the subject 104 during the second time interval. The parameter analyzer 112 may determine that the blood pressure of the subject 104 is rising during the second time interval. Based on determining that the blood pressure is rising, the parameter analyzer 112 may determine ROSC has occurred. In some instances, the parameter analyzer 112 may determine a third time interval during which the treatment is not being administered. The parameter analyzer 112 may be configured to determine a change in the physiological parameter (e.g., the blood pressure) between the second time interval and the third time interval. The parameter analyzer 112 may determine that the blood pressure of the subject 104 is higher during the third time interval than during the second time interval. Based on determining that the blood pressure is higher, the parameter analyzer 112 may determine ROSC has occurred. In some examples, the parameter analyzer 112 is configured to analyze an ECG of the subject 104 during the second time interval. The parameter analyzer 112 may be configured to determine a shape, a duration, an axis, a timing relative to the P wave, or another characteristic of the QRS complex. Based on analyzing a QRS complex in the ECG, the parameter analyzer 112 may determine that the subject 104 has an arrythmia (e.g., ventricular tachycardia). In some examples, the parameter analyzer 112 is configured to modify or temporally gate the detection of the physiological parameter based on analyzing the treatment parameter. The parameter analyzer 112 may transmit a signal to the first sensor 106 indicative of whether treatment is being administered. Based on receiving the signal, the first sensor 106 may be configured to detect the physiological parameter and transmit data to the parameter analyzer 112 indicative of the physiological parameter.

In some cases, the parameter analyzer 112 is configured to validate the physiological parameter based on the signal detected by the second sensor 108. For example, the parameter analyzer 112 may compare the signals detected by the first sensor 106 and by the second sensor 108. The parameter analyzer 112, in some cases, may determine that the first sensor 106 is not functioning properly. For example, if an ECG detected by the first sensor 106 indicates that a heart of the subject 104 is beating but a blood flow detected by the second sensor 108 indicates that blood is not circulating in the subject 104, the parameter analyzer 112 may determine that the second sensor 108 may be malfunctioning. In some cases, the parameter analyzer 112 may determine a confidence level associated with the physiological parameter based on comparing the signals detected by the first sensor 106 and the second sensor 108. In some examples, the parameter analyzer 112 determines that the first sensor 106 is more reliable or accurate than the second sensor 108. For example, based on determining that the first sensor 106 is more reliable than the second sensor 108, the parameter analyzer 112 may determine that the second sensor 108 is malfunctioning. In some cases, based on determining that the first sensor 106 is more reliable, the parameter analyzer 112 may weight the signal detected by the first sensor 106 higher than the signal detected by the second sensor 108. Based on weighting the signals detected by the first sensor 106 and the second sensor 108, the parameter analyzer 112 may determine a confidence level associated with the physiological parameter. In some instances, the parameter analyzer 112 is configured to output an indication to the user 110 that the first sensor 106 is malfunctioning. In some examples, the parameter analyzer 112 may guide a user to correctly adhere the first sensor 106 to the subject 104 or to correct the position of the first sensor 106.

According to various implementations, the parameter analyzer 112 may provide feedback about a treatment being administered to the subject 104 or a condition of the subject 104. For example, based on filtering and/or temporally gating the physiological parameter, the parameter analyzer 112 may determine that the treatment of the subject 104 should be adjusted in order to maximize the efficacy of the treatment. In some implementations, the treatment device 102 is administering chest compressions to the subject 104, and the parameter analyzer 112 may direct the user 110 to change a position, frequency, duty cycle, or depth of chest compressions administered to the subject 104, or to pause or resume chest compressions.

In some implementations, the parameter analyzer 112 is connected to the treatment device 102. The treatment device 102 may transmit, to the parameter analyzer 112, a signal indicating a time at which the treatment device 102 is administering the treatment to the subject 104. In some examples, the treatment device 102 is a mechanical chest compression device. The treatment device 102 may transmit a signal to the parameter analyzer 112 indicating a position, frequency, duty cycle, or depth of chest compressions. According to various implementations, the parameter analyzer 112 is configured to filter or gate the physiological parameter based on the signal transmitted by the treatment device 102. In some examples, the parameter analyzer 112 may direct the treatment device 102 to change a position, frequency, duty cycle, or depth of chest compressions administered to the subject 104, or to pause or resume chest compressions.

In some examples, the parameter analyzer 112 may be configured to temporally gate or disconnect the first sensor 106 based on communication signals received from the treatment device 102 indicative of a treatment to be administered. For instance, the treatment device 102 may include an automated external defibrillator (AED). The parameter analyzer 112 may transmit a signal to the first sensor 106 indicating that an electrical shock will be administered to the subject 104 at a particular time. The first sensor 106 may include a circuit with at least one switch that disconnects or otherwise shields sensitive electronics in the first sensor 106 during the treatment. In response to receiving the signal, the first sensor 106 may be configured to disconnect or shield the sensitive electronics.

The external device 116 is configured to transmit communication signals 118 with the parameter analyzer 112 to assist with the care of the subject 104. In some implementations, the external device 116 is operated by a clinician or other healthcare professional. The external device 116 may enable the clinician to provide clinical guidance to the user 110. In various cases, the external device 116 is configured to communicate or provide alerts to emergency medical personnel. Alerts include, in various implementations, an audio alert, a visual alert (e.g., a push notification, a SMS/MMS message, an Over-the-Top (OTT) message, an in-application alert, or the like), haptic feedback (e.g., vibrations), or the like. In some examples, the external device 116 is configured to store data indicative of the treatment administered to the subject 104 and/or the condition of the subject 104.

In particular implementations, the treatment device 102 is a mechanical chest compression device which is administering chest compressions to the subject 104. The first sensor 106 may be disposed on the subject 104 to determine an efficacy of the chest compressions. In some examples, the first sensor 106 includes an ultrasound transducer configured to detect a blood flow of the subject 104. The ultrasound transducer may be disposed on the skin the of subject 104 and emit ultrasound waves towards a blood vessel(s) of the subject 104. The ultrasound transducer may detect a reflection of the ultrasound waves in order to determine a velocity of blood flow in the blood vessel(s) by Doppler processing of the reflected ultrasound waves. In some examples, the second sensor 108 is an accelerometer configured to detect an acceleration of the subject 104. The second sensor 108 may be disposed on the subject 104 and detect acceleration of the subject 104 during a chest compression. The first sensor 106 and the second sensor 108, in various examples, transmit signals indicative of the blood flow of the subject 104 and the chest compressions, respectively, to the parameter analyzer 112. Based on receiving the signal indicative of chest compressions, the parameter analyzer 112 may determine an artifact associated with the chest compressions. In some examples, the parameter analyzer 112 determines a frequency of chest compressions (e.g., a compression frequency). The parameter analyzer 112, in some examples, designs a filter to attenuate the compression frequency and applies the filter to the signal indicative of the blood flow. The filter, in some examples, is a notch filter or a band reject filter. In some cases, the parameter analyzer 112 designs a filter to attenuate an artifact associated with the chest compressions and applies the filter to the signal indicative of the blood flow (i.e., a time domain representation of the signal indicative of the blood flow). Based on applying the filter to the signal indicative of the blood flow, the parameter analyzer 112 may analyze the filtered signal indicative of the blood flow to determine a velocity of blood flow in the subject 104. In some examples, the parameter analyzer 112 communicates the blood flow velocity to the user 110 or the external device 116.

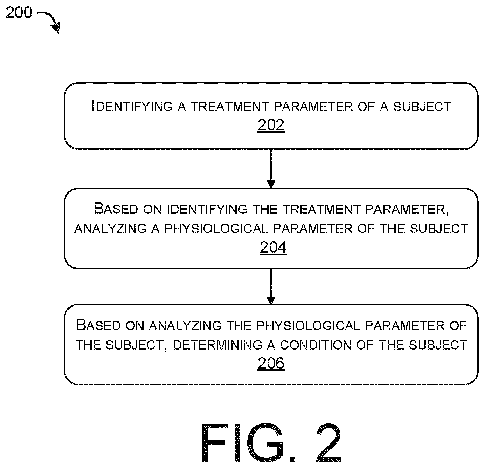

FIG. 2 illustrates an example method 200 for determining a condition of a subject (e.g., the subject 104). According to some implementations, the method 200 is performed by an entity, such as a sensor (e.g., the first sensor 106 or the second sensor 108), medical device (e.g., the treatment device 102), computing device, at least one processor, or a combination thereof.

At 202, the entity identifies a treatment artifact associated with a treatment administered to the subject (e.g., the subject 104). In some examples, the entity receives a signal indicative of a treatment parameter from a sensor (e.g., the second sensor 108) or a medical device treating the subject (e.g., the treatment device 102). The treatment, in various instances, may include chest compressions, artificial ventilation, defibrillation, or another treatment administered to the subject. The entity may analyze the signal to determine the treatment artifact. In some examples, the treatment parameter is a frequency, depth, location, or time period of chest compressions. In various instances, the treatment parameter is a frequency, an energy level, or a time period of electrical shocks delivered to the subject by a defibrillator.

At 204, the entity analyzes a physiological parameter based on the treatment parameter. In some examples, the entity receives a second signal indicative of the physiological parameter from a second sensor (e.g., the first sensor 106). The second sensor may be configured to detect an ECG, an impedance, a force administered to the patient, a blood pressure, an airway parameter, a blood oxygenation, an electroencephalogram, a temperature, a heart sound, a blood flow rate, a physiological geometry, a heart rate, a pulse rate, or another type of metric indicative of a condition of the subject. In some cases, the entity receives the second signal from a medical device (e.g., the treatment device 102). Based on the treatment parameter, the entity may, in some examples, filter and/or temporally gate the measurement of the physiological parameter. For instance, the entity may identify, based on the first signal, a first time during which treatment is being administered and a second time during which treatment is paused. Based on identifying the first time and the second time, the entity may determine, in some examples, that the treatment interferes with the detection of the physiological parameter, and the entity may analyze the physiological parameter during the second time. In some cases, the entity is configured to identify and remove an artifact associated with the treatment (e.g., a treatment artifact) from the physiological parameter. For example, the entity may remove a treatment artifact from the blood flow by applying a band reject filter centered around the chest compression frequency or by applying a comb filter that includes the chest compression frequency and one or more harmonics of the chest compression frequency. In various examples, the entity outputs a first indication of the physiological parameter during the first time interval and a second indication of the physiological parameter during the second time interval. The first indication and the second indication may be distinguished by color, style, or another display metric.

At 206, the entity determines a condition of the subject based on analyzing the physiological parameter. For example, the entity may determine the condition of the subject (e.g., a pulse, a blood flow rate, an ECG, or another physiological parameter) based on removing the treatment artifact from the physiological parameter. In some examples, the entity determines the condition of the subject by temporally gating the measurement of the physiological parameter. For instance, the entity may determine a pulse of the subject by analyzing the physiological parameter during the second time. The entity, in some implementations, is configured to communicate the condition of the subject to an external device (e.g., the external device 116), a user (e.g., the user 110), or a medical device (e.g., the treatment device 102, the first sensor 106, or the second sensor 108). For instance, the entity may output an indication to the user that the condition of the subject is stable, worsening, or improving. In some instances, the entity determines that the treatment should be adjusted based on determining the condition of the subject. For example, the entity may determine that a frequency or a depth of chest compressions based on determining that a blood oxygenation of the subject is lower than a recommended range. In some cases, the entity may identify ROSC and determine that chest compressions should be stopped.

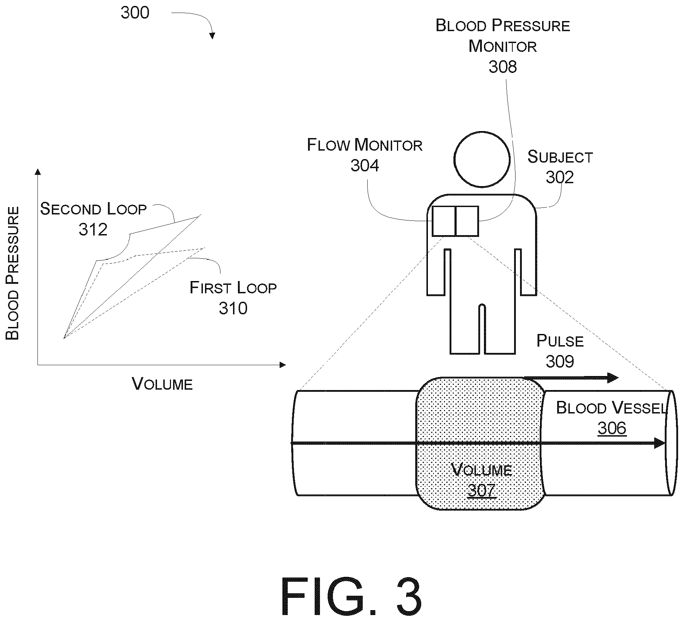

FIG. 3 illustrates an example of an environment 300 in which a condition of a subject 302 is evaluated based on a blood pressure and a blood flow parameter of the subject 302. As shown, a flow monitor 304 detects a blood flow through a blood vessel 306 of the subject 302. Simultaneously, a blood pressure monitor 308 detects a blood pressure through the blood vessel 306. Although illustrated as separate devices in FIG. 3, implementations are not so limited. For example, in some cases, the flow monitor 304 and the blood pressure monitor 308 are integrated into a single device (e.g., with a single housing). In some cases, the flow monitor 304 and/or the blood pressure monitor 304 include at least one of the first sensor 106, the second sensor 108, or the parameter analyzer 112.

In various implementations, the flow monitor 304 is configured to detect a volume 307 of a portion of the blood vessel 306 of the subject. The volume 307 is defined between two cross-sections of the blood vessel 306, for instance. As a pulse 309 travels along the blood vessel 306, the wall of the blood vessel 306 may stretch such that the volume 307 temporarily expands. After the pulse 309 has passed the portion of the blood vessel 306, the volume 307 is temporarily reduced.

The volume 307 is detected, or estimated, by the flow monitor 304 using one or more techniques. For example, the flow monitor 304 detects a velocity profile of blood flowing through the blood vessel 306, using one or more Doppler-based techniques described herein, and may estimate the volume 307 based on the velocity profile. In some cases, the flow monitor 304 estimates the volumetric flow rate through the first cross section on one side of the volume 307 and the volumetric flow rate through the second cross section on the other side of the volume 307, and estimates the volume 307 based on a difference between the volumetric flow rates. For instance, the volume 307 may be calculated by integrating the difference between the volumetric flow rates with respect to time. Volumetric flow rate through a cross-sectional area, for instance, can be estimated by integrating the velocity profile over the cross-sectional area. The flow monitor 304, for instance, samples the volume 307 at a sampling rate.

According to some examples, the blood pressure monitor 308 is configured to detect a blood pressure in the blood vessel 306 of the subject. In some cases, the blood pressure monitor 308 invasively detects the blood pressure via a catheter inserted into the blood vessel 306. In some examples, the blood pressure monitor 308 is a noninvasive device. Examples of noninvasive devices configured to detect blood pressure include, for instance, blood pressure cuffs and/or wearable devices (e.g., watches) configured to use plethysmography and/or pulse transit time to infer blood pressure.

In some examples, the blood pressure monitor 308 is a noninvasive device that estimates blood pressure by detecting parameters of the blood vessel 306. For example, an instantaneous measurement of blood pressure (also referred to as "pulse pressure") can be derived based on a blood velocity through the blood vessel 306, a velocity of the pulse 309 traveling down the blood vessel 306 (e.g., a pulse wave velocity (PWV)), and a density of blood in the blood vessel 306 via the Water Hammer Equation. Alternatively, an instantaneous measurement of blood pressure (e.g., pulse pressure) can be derived based on a blood velocity through the blood vessel 306 and a pulse wave velocity measurement of the blood vessel 306 wall by means of a lookup table or mathematical model configured based upon empirical measurements. Blood velocity can be noninvasively detected via various techniques described herein. The PWV can be detected by detecting a time delay between a movement of a first portion of the wall of the blood vessel 306 and a movement of a second portion of the wall of the blood vessel 306, divided by a distance between the first portion of the wall and the second portion of the wall. According to some implementations, the blood pressure monitor 308 detects the movement at the first and second portion of the wall of the blood vessel 306 by detecting a Doppler shift between an incident beam directed toward wall of the blood vessel 306 and a return beam from the wall of the blood vessel 306. The beams, for instance, may include ultrasound and/or light. In various cases, the viscosity of blood may be derived based on population-based features. For instance, the density of the blood in the blood vessel 306 may be equivalent to an average blood density of a population (e.g., 1,060 kg/m³). Examples of devices and techniques for detecting blood pressure are described, for instance, in U.S. Pub. No. 2018/0199834, U.S. Pub. No. 2018/0369065, U.S. Pub. No. 2018/0368804, U.S. Pub. 2019/0046152, U.S. Pub. No. 2019/0053779, U.S. Pub. No. 2019/0125191.

The blood pressure monitor 308, in various implementations, detects the blood pressure of the blood vessel 306 over time. For instance, the blood pressure monitor 308 detects an instantaneous blood pressure in the blood vessel 306 at a sampling rate.

In various cases, the blood pressure detected by the blood pressure monitor 308 is not necessarily accurate. In some cases, the blood pressure monitor 308 detects and reports a metric that is proportional to the blood pressure of the subject 302, without being accurately representative of the blood pressure of the subject 302, using any techniques described herein. As used with respect to the description of at least FIG. 3, the term "blood pressure" may refer to an accurate blood pressure or any metric that is proportional or otherwise related to the blood pressure. In various implementations, the flow monitor 304 receives an indication of the blood pressure with respect to time from the blood pressure monitor 308.

According to various implementations of the present disclosure, the flow monitor 304 detects a condition of the subject 302 and/or monitors a treatment to the subject 302, by analyzing a relationship between the volume 307 and the blood pressure of the blood vessel 306. In some implementations, the volume 307 and the blood pressure each vary during a cardiac cycle (e.g., a cycle of diastole and systole). Assuming a closed system (e.g., the subject 302 does not have significant bleeding), the volume 307 (and volumetric flow rate) increases as blood pressure increases, and falls and blood pressure falls. As illustrated in FIG. 3, if the volume 307 and blood pressure measurements during a single cardiac cycle are plotted on a Cartesian coordinate system, the measurements form a first loop 310. The flow rate and blood pressure measurements during a subsequent cardiac cycle are represented by a second loop 312. The first loop 310 and the second loop 312, for instance, are pressure-volume (PV) loops. For example, a slope (e.g., a peak or mean slope) of the measurements during diastole may be different than a slope (e.g., a peak or mean slope) of the measurements during systole. In some implementations, the flow monitor 304 outputs an indication of the first loop 310 and/or the second loop 312. In some cases, the flow monitor 304 outputs an indication of one or more slopes of the first loop 310 and/or the second loop 312.

According to some examples, the flow monitor 304 includes a display that visually presents the first loop 310 and the second loop 312 to a rescuer. The rescuer, therefore, can assess a condition of the subject 302 by reviewing the first loop 310 and the second loop 312. In some cases, the first loop 310 and the second loop 312 are displayed simultaneously by the flow monitor 304. To differentiate between the first loop 310 (e.g., the older PV loop) and the second loop 312 (e.g., the more recent PV loop), the first loop 310 and the second loop 312 may be displayed with different characteristics. For instance, the first loop 310 may have a different color, a thinner line thickness, a greater transparency value, or a different line style (e.g., dotted or dashed versus solid) than the second loop 312. In various cases, the flow monitor 304 displays additional loops, or replaces the first loop 310 and/or the second loop 312 with additional loops, as they are detected from the subject 302.

The flow monitor 304, in various implementations, detects a change in a compliance of the blood vessel 306 by detecting a change in a slope (e.g., peak or mean, diastolic or systolic, etc.) of the first loop 310 with respect to a slope of the second loop 312 (e.g., peak or mean, diastolic or systolic, etc.). The slopes may be defined during the same type of phase of different cyclical events. For example, the slopes may be defined during systole or diastole of different cardiac cycles of the subject 302. In some cases, the slopes may be defined during compression or release of different chest compressions performed on the subject 302. The term "compliance" may refer to a characteristic of a blood vessel to expand in response to increasing internal pressure of the blood in the blood vessel. Compliance, for instance, can be estimated based on the slope of the first loop 310 or the slope of the second loop 312.

If a difference between the slope of the first loop 310 and the slope of the second loop 312 exceeds a first threshold (e.g., the slope significantly increases) or is below a second threshold (e.g., the slope significantly decreases), the flow monitor 304 may perform one or more actions. According to some cases, the flow monitor 304 outputs an indication of the change in compliance. A change in compliance in the blood vessel 306 may be caused by the delivery of a medication, such as epinephrine or vasopressin, which increases vascular stiffness. Thus, in some cases, the flow monitor 304 detects when the subject 302 has been dosed with a medication in response to detecting that the difference between the slope of the first loop 310 and the slope of the second loop 312 is below the second threshold. The flow monitor 304, for instance, may output an indication that the subject 302 is predicted to have been dosed with a medication to a user or may store an indication of the time at which the subject 302 is predicted to have been dosed with the medication in an electronic medical record associated with the subject 302.

In some cases, a compliance of the subject 302 is indicative of a condition of the subject 302. For instance, compliance may be indicative of decompensation, diabetes, compression syndrome, or other adverse medical conditions. In some implementations, the flow monitor 304 detects a condition of the subject 302 by determining that a difference between the slope of the first loop 310 or the slope of the second loop 312 exceeds a first threshold or is less than a second threshold. The flow monitor 304, in various cases, outputs an indication that the subject 302 is suspected to have the condition.

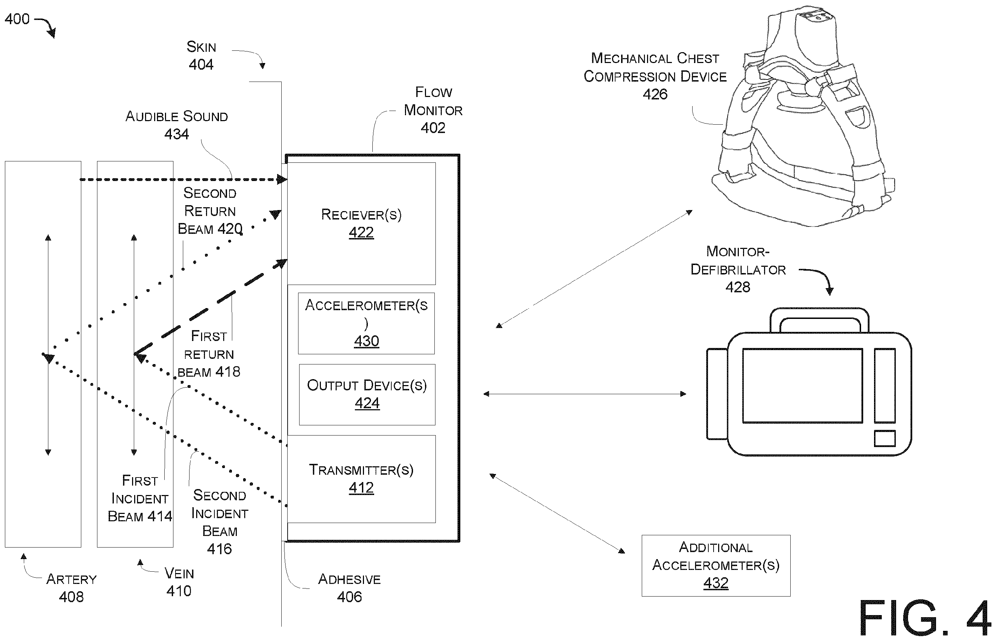

FIG. 4 illustrates an example environment 400 for monitoring blood flow through one or more blood vessels of a subject. A flow monitor 402 is adhered to skin 404 of the subject via an adhesive 406. In some examples, the flow monitor 402 includes at least one of the first sensor 106, the second sensor 108, the parameter analyzer 112, or the flow monitor 304. The flow monitor 402, for instance, is located outside of the body of the subject. In some implementations, the flow monitor 402 is held on the skin 404 by a strap, a buckle, a bandage, or some other fastener. For instance, the flow monitor 402 may be wrapped around an extremity of the subject.

In various implementations, an artery 408 and a vein 410 are disposed underneath the skin 404. The artery 408 and the vein 410 are part of the circulatory system of the subject. The circulatory system includes a fluid circuit of various blood vessels (including the artery 408 and the vein 410). The subject includes a heart that, when functioning, pumps blood through the blood vessels. In particular, the heart moves blood from lungs of the subject, where the blood can be oxygenated, to other portions of the subject's body, such as the brain, other organs, and the subject's extremities. Along the circulatory system, cells within the blood deliver oxygen to cells of the subject, thereby supporting cellular respiration. In various cases, the artery 408 carries oxygenated blood from the lungs of the subject and the vein 410 carries deoxygenated blood toward the lungs. Examples of the artery 408 include a carotid artery, a subclavian artery, a coronary artery, a brachial artery, an iliac artery, a radial artery, a femoral artery, or a pulmonary artery. Examples of the vein 410 include a jugular vein, an iliac vein, a subclavian vein, a cephalic vein, a brachial vein, a basilic vein, a hepatic vein, a radial vein, an ulnar vein, a digital vein, a brachiocephalic vein, a femoral vein, a saphenous vein, a venous arch, or a tibial vein. In some cases, the pulmonary artery carries deoxygenated blood.

According to various implementations of the present disclosure, the flow monitor 402 is configured to detect the flow of blood through the artery 408 and/or vein 410. In particular cases, the flow monitor 402 includes one or more transmitters 412 configured to output one or more incident beams toward the artery 408 and/or vein 410. In the example illustrated in FIG. 4, the incident beams include a first incident beam 414 and a second incident beam 416.

In various cases, the first incident beam 414 and the second incident beam 416 include waves that are transmitted through the skin 404. The waves, for example, can be instantiated as light and/or sound. In various cases, the first incident beam 414 and the second incident beam 416 include at least one of infrared, near-infrared, or visible light. For instance, the light may have a frequency in a range of 300 GHz - 430 THz and a wavelength in a range of 700 nanometers (nm) to 1 millimeter (mm). For instance, the transmitter(s) 412 include one or more light sources, such as light-emitting diodes (LEDs) or lasers. In some examples, the transmitter(s) 412 may include one or more mirrors configured to split the light output by the light source(s), such as for an interferometric analysis. In some cases, the receiver(s) 422 include one or more light sensors, such as at least one of a photodiode, a phototransistor, a photomultiplier tube, a charge-coupled device, a metal-semiconductor-metal photodetector, or a complementary metal oxide semiconductor photodetector.

According to various implementations, the waves include ultrasound. As used herein, the term "ultrasound," and its equivalents, can refer to mechanical waves (e.g., in the form of pressure waves) having a frequency in a range of 20 kilohertz (kHz) to 200 megahertz (MHz). Ultrasound, for example, is sound in a frequency that is greater than an upper detection limit of a human ear. In various instances, transmitter(s) 412 include one or more piezoelectric crystals (including, e.g., lead zirconate titanate (PZT), LiNbO₃ (LN), lead magnesium niobate-lead titanate (PMN-PT), or lead indium niobate- lead magnesium niobate-lead titanate (PIN-PMN-PT)). When an electrical current is induced through the piezoelectric crystal(s), the piezoelectric crystal(s) vibrate at a frequency that produces ultrasound. In some examples, the transmitter(s) 412 include one or more micro-electromechanical system (MEMS) devices. In some instances, the transmitter(s) 412 include one or more capacitive micromachined ultrasonic transducers (CMUTs) and/or piezoelectric micromachined ultrasonic transducers (PMUT). Any electrical to mechanical conversion system or material that operates at the ultrasound frequency range would be suitable.

According to various implementations, the flow monitor 402 includes one or more ultrasound transducers. As used herein, the terms "ultrasound transducer," "ultrasonic transducer," "transducer," and their equivalents, may refer to a device that generates or detects ultrasound. For instance, the ultrasound transducer(s) include the transmitter(s) 412 and/or the receiver(s) 422. In some cases, an ultrasound transducer includes a transducer element (e.g., a piezoelectric crystal, MEMS device, or another type of electrical-to-mechanical conversion device), a first electrode disposed on one side of the transducer element, and a second electrode disposed on another side of the transducer element. In various implementations, the ultrasound transducer is configured to produce ultrasound by inducing a current through or a voltage between the first and second electrodes. In some cases, the ultrasound transducer is configured to detect ultrasound by detecting a current through or voltage between the first and second electrodes that is induced when the ultrasound is received by the transducer element. In some cases, the ultrasound transducer is encased in a housing, which may be watertight. The ultrasound transducer, in some examples, further includes a matching layer that is disposed between the transducer element and a surface of the housing from which an incident beam of ultrasound is emitted. The matching layer includes a material having an acoustic impedance that is between the acoustic impedance of the transducer element and an acoustic lens (if one exists or between the transducer and skin). In some implementations, a gel layer is disposed between the housing of the ultrasound transducer and the skin 404 that further matches the impedance between the ultrasound transducer and the body, thereby preventing the first incident beam 414 and the second incident beam 416 from being reflected by an interface containing air between the skin 404 and the ultrasound transducer. In some cases, the adhesive 406 serves as the gel layer.

In some examples, the first incident beam 414 is reflected and/or scattered by blood in the vein 410, thereby generating a first return beam 418. Further, in some cases, the second incident beam 416 is reflected and/or scattered by blood in the artery 408, thereby generating a second return beam 420. The flow monitor 402 includes one or more receivers 422 configured to detect the first return beam 418 and/or the second return beam 420.

As illustrated, the artery 408 and the vein 410 are substantially parallel to the skin 404. In various implementations of the present disclosure, the transmitter(s) 412 emit the first incident beam 414 and/or the second incident beam 416 in a direction that is non-perpendicular and non-parallel to the surface of the flow monitor 402 that is adhered to the skin 404. That is, the transmitter(s) 412 emit the first incident beam 414 and/or the second incident beam 416 in an angled fashion. Thus, a component of the first incident beam 414 is parallel to the blood flow through the vein 410 and/or a component of the second incident beam 416 is parallel to the blood flow through the artery 408.

Various implementations of the transmitter(s) 412 that enable angled transmission of the first incident beam 414 and the second incident beam 416 are disclosed herein. In some implementations in which the transmitter(s) 412 include one or more transducer elements configured to emit ultrasound, an individual transducer element may have the shape of a cylinder (with a height of the cylinder being substantially parallel to the skin 404 and perpendicular to a direction of the artery 408 and/or vein 410) or a polygonal prism (with a height of the polygonal prism being substantially parallel to the skin 404 and perpendicular to a direction of the artery 408 and/or vein 410). In some cases, a piezoelectric crystal may be divided into sections defined by an angle perpendicular to the height of the cylinder, each section serving as a different transducer element configured to emit ultrasound. In some cases, the transmitter(s) 412 include multiple (e.g., planar) transducer elements that emit respective ultrasound beams that are phase networked together such that they collectively form the angled first incident beam 414 or the second incident beam 416. In some cases, the transmitter(s) 412 include a single transducer element including portions that are insensitive to ultrasound (e.g., non-piezoelectric portions, such as including a polymer) and portions that are sensitive to ultrasound (e.g., piezoelectric portions), which can cause the first incident beam 414 or the second incident beam 416 to be output as a grating lobe. The grating lobe may include components emitted at different angles, at least one of which may include the first incident beam 414 or the second incident beam 416 to be transmitted at an angle with respect to the artery 408 or vein 410.

In some implementations, the transmitter(s) 412 include a transducer element that emits ultrasound from a surface that is nonparallel to the skin 404. For example, the flow monitor 402, in some cases, includes a wedge-shaped spacer between the crystal and the skin 404. The spacer may be substantially acoustically transparent. In some examples, the wedge-shaped spacer includes a polymer or gel that is configured to perform impedance matching between the crystal and the skin 404. For instance, the spacer may include multiple layers arranged in steps that are configured to be in contact with the crystal and/or tilt the crystal with respect to the skin 404.

In some cases, the flow monitor 402 includes an acoustic "lens" that is disposed between a crystal emitting ultrasound and the skin 404. For example, the acoustic lens can be a spacer with a nonuniform acoustic impedance. Thus, the acoustic lens may bend the ultrasound emitted by the crystal before it is transmitted through the skin 404.

In some examples, the transmitter(s) 412 includes a crystal that is disposed inside of a needle that is disposed through the skin 404. In some implementations, the transmitter(s) 412 include an array of crystals configured to emit the first incident beam 414 and/or the second incident beam 416 through the skin 404. For example, the array may be arranged on a curved surface, such that individual crystals may point in different directions. In operation, the flow monitor 402 may automatically determine which incident beams whose return beams produce a greatest frequency and/or phase shift with respect to the incident beams, and may define those beams as the first incident beam 414 and/or the second incident beam 416.

In various implementations, the first return beam 418 may represent a frequency and/or phase shift with respect to the first incident beam 414 due to the Doppler effect. Similarly, because the component of the second incident beam 416 is parallel to the blood flow through the artery 408, the second return beam 420 may represent a frequency and/or phase shift with respect to the second incident beam 416 due to the Doppler effect. These shifts occur due to the Doppler effect and may be referred to as "Doppler shifts."

According to various implementations, the flow monitor 402 determines a velocity of the blood flow through the artery 408 and the vein 410 due to a difference between the frequencies of the first incident beam 414 and the first return beam 418, as well as a difference between the frequencies of the second incident beam 416 and the second return beam 420. The transmitter(s) 412, in some cases, operate in a continuous wave Doppler mode (also referred to as "CW Doppler"), and continuously transmit the first incident beam 414 and the second incident beam 416 during a monitoring period. The receiver(s) 422, for instance, continuously detect the first return beam 418 and the second return beam 420 during the monitoring period. For instance, the flow monitor 402 detects the blood velocity in the artery 408 and the blood velocity in the vein 410, in-real time, continuously or semi-continuously based on the frequency shifts between the first incident beam 414 and the first return beam 418 as well as between the second incident beam 416 and the second return beam 420.

In various implementations, the flow monitor 402 operates in a pulsed-wave Doppler mode (also referred to as "PW Doppler"). For instance, the flow monitor 402 causes the incident beam 416 to output the first incident beam 414 and/or the second incident beam 416 in pulses and detects the first return beam 418 and/or the second return beam 420 as return pulses. In various cases, a time delay between the output pulses and the return pulses is indicative of the depth of a structure from which the return pulses are reflected. In various implementations, the flow monitor 402 can determine the blood velocity in the artery 408 based on phase shifts between the pulses of the first incident beam 414 and the first return beam 418. Further, the flow monitor 402 can determine the blood velocity in the vein 410 based on phase shifts between the pulses of the second incident beam 416 and the second return beam 420.

In some cases, the flow monitor 402 detects the blood velocities at a sampling rate that corresponds to at least twice the component of the velocity range in the direction of the beam pointing angle of the first incident beam 414 and/or the second incident beam 416.

In some examples, the flow monitor 402 performs laser Doppler velocimetry in order to detect the blood velocity. In various cases, the flow monitor 402 includes one or more interferometric sensors. For example, the first incident beam 414 and the second incident beam 416 are light beams (e.g., coherent light beams) that are split (e.g., by one or more mirrors) prior to transmission through the skin 404. The flow monitor 402 may detect the blood velocities in the artery 108 and the vein 410 by comparing the first return beam 418 and the second return beam 420 to the split beams generated from the first incident beam 414 and the second incident beam 416. Techniques for interferometric detection of blood velocity can be found in, for example, R. D. Rader, C. M. Stevens and J. P. Meehan, "An Interferometric Blood Flow Measurement Technique - A Brief Analysis," in IEEE Transactions on Biomedical Engineering, vol. BME-21, no. 4, pp. 293-297, July 1974; Nagahara, et al., Method. Invest. Ophthalmol. Vis. Sci. 2011;52(1):87-92; and Robinson, et al., Sci Rep 13, 8803 (2023).

In some cases, the flow monitor 402 images a portion of the subject that includes the artery 408 and the vein 410. In some cases, the flow monitor 402 generates an image using the first return beam 418 and the second return beam 420 using one or more sonographic techniques. In various implementations, the flow monitor 402 generates the image using multiple return beams including the first return beam 418 and the second return beam 420. The flow monitor 402, in some implementations, generates the multiple return beams by sweeping the first incident beam 414 and the second incident beam 416 across a section of the subject being imaged. For example, the flow monitor 402 may generate a real-time image of the subject that includes cross-sections of the artery 408 and vein 410, respectively. In some implementations, the flow monitor 402 automatically segments the cross-sections of the artery 408 and vein 410 in the real-time image. In some cases, the flow monitor 402 performs segmentation of a scrolling Doppler image (e.g., Doppler shift in a y-axis is swept in time along an x-axis) to segregate out the Doppler information of the vein 410 from the artery 408. Various types of segmentation techniques can be used, such as detection using histogram of oriented gradients (HOG) features, a scale-invariant feature transform (SIFT), Viola-Jones object detection framework, or You Only Look Once (YOLO). Once the cross-sections depicted in the image are identified using image segmentation, the flow monitor 402 can further classify the cross-sections using a support vector machine (SVM). In some cases, the flow monitor 402 uses one or more trained convolutional neural networks (CNNs) to segment and/or classify the cross-sections of the artery 408 and the vein 410 depicted in the image. In various cases, the flow monitor 402 may differentiate the cross-section corresponding to the artery 408 an the cross-section corresponding to the vein 410.

In particular implementations, the flow monitor 402 detects the velocity of the blood through the artery 408 and the velocity of the blood through the vein 410, simultaneously. For example, the first return beam 418 may be reflected from the artery 408 and the second return beam 420 may be reflected from the vein 410, so that the flow monitor 402 can detect the blood velocity of the artery 408 based on the first return beam 418 and may detect the blood velocity of the vein 410 based on the second return beam 420.

Simultaneously monitoring the blood velocity through the artery 408 and the vein 410 may enable certain evaluations of the subject. In some implementations, the flow monitor 402 detects a volumetric flow rate or net flow volume (e.g., during a time period) through at least a portion of the subject based on the blood velocity through the artery 408 and the vein 410. In some cases, the flow monitor 402 determines the volumetric flow rate through the artery 408 by integrating the velocity of the blood through the artery 408 across a cross-sectional area of the artery 408. The flow monitor 402 may determine the volumetric flow rate through the vein 410 by integrating the velocity of the blood through the vein 410 across a cross-sectional area of the vein 410. In various implementations, the flow monitor 402 is configured to detect a net flow volume that flows through the cross-section of the artery 408 during a time period (e.g., a cardiac cycle, a chest compression cycle, a portion of a chest compression cycle, or any combination thereof) by integrating the volumetric flow rate through the artery 408 over the time period. Similarly, the flow monitor 402 is configured to detect a net flow volume that flows through the cross-section of the vein 410 during the time period by integrating the volumetric flow rate through the vein 410 over the time period.

The artery 408, for instance, supplies oxygenated blood to the portion of the subject, and the vein 410 transports deoxygenated blood from the portion of the subject. In some examples, the flow monitor 402 is configured to detect a net flow volume of blood to a portion of the subject's body over time. In particular cases, the artery 408 is a carotid artery and the vein 410 is a jugular vein, and the flow monitor 402 is configured to detect a net flow volume of blood to the brain of the subject over time.

The flow monitor 402 may assess a condition of the subject and/or evaluate a treatment administered to the subject by analyzing blood flow parameters (e.g., velocity, volumetric flow rate, or net flow volume of blood) in the artery 408 and/or the vein 410. In some implementations, the flow monitor 402 identifies chest compressions performed on the subject based on the blood velocity through the artery 408 and/or the blood velocity through the vein 410. When (e.g., effective) chest compressions are performed on the subject, the compressions push blood through the artery 408 and the vein 410 in a direction that moves distally from the chest. Thus, chest compressions cause blood flowing through the artery 408 and blood flowing through the vein 410 to travel in parallel directions. Moreover, an individual chest compression causes the blood to flow in the artery 408 and vein 410 in a surge. Thus, in some cases, the flow monitor 402 detects that chest compressions are being performed on the subject by detecting that the blood through the artery 408 and the blood through the vein 410 is traveling in the same or parallel directions (e.g., in a direction pointing distally from the chest). For instance, the flow monitor 402 can detect whether chest compressions are undesirably moving blood in the same direction through the artery 408 and the vein 410.

In some cases, the flow monitor 402 provides feedback about the efficacy of the chest compressions based on the blood velocity through the artery 408 and the vein 410 and/or the net flow volume to the portion of the subject. For instance, if the flow monitor 402 detects less than a threshold blood velocity through the artery 408 or vein 410, or detects less than a threshold net flow volume to the portion of the subject (e.g., during a particular time period), the flow monitor 402 can output a feedback signal via one or more output devices 424. The output device(s) 424, for instance, include a display (e.g., a screen configured to visually output signals), a speaker (e.g., configured to audibly output signals), a haptic feedback device (e.g., configured to convey signals by vibrating or otherwise moving), a transceiver (e.g., configured to transmit signals to external devices), or any combination thereof. In some cases, the feedback signal is output to a rescuer (not illustrated) performing the chest compressions, and may cause the rescuer to adjust the depth of the chest compressions, adjust the frequency of the chest compressions, or adjust the position of the chest compressions relative to the subject's chest.

In some implementations, the feedback signal is transmitted to a mechanical chest compression device 426 that is performing the chest compressions. The mechanical chest compression device 426, in some cases, administers the chest compressions by moving a plunger up and down on the subject's chest. The feedback signal, for instance, causes the mechanical chest compression device 426 to adjust the depth of the chest compressions, adjust the frequency of the chest compressions, adjust the position of the plunger relative to the subject's chest, pause chest compressions, or initiate chest compressions. In some implementations, the mechanical chest compression device 426 transmits a signal to the flow monitor 402 that indicates the timing, frequency, position, or another chest compression parameter characterizing the chest compressions administered by the mechanical chest compression device 426. The flow monitor 402, in some cases, generates the feedback signal based on the chest compression parameter, such that the feedback signal is specific to the conditions reported by the mechanical chest compression device 426.

The placement of chest compressions, in particular, impacts the flow of blood through specific blood vessels through the subject's body. In some cases, the flow monitor 402 enables optimization of the position of the chest compressions based on the flow rate through the artery 408, the flow rate through the vein 410, the net flow volume through the portion of the subject's body, a current position of the chest compressions, an identity or position of the artery 408 in the subject's body, an identity or position of the vein 410 in the subject's body, or any combination thereof. For example, if the chest compressions are being administered to the right of an ideal position, the flow monitor 402 may detect a peak and/or mean flow rate of blood in the artery 408 that is below a threshold. In various implementations, the flow monitor 402 (or another computing device that is communicatively coupled to the flow monitor 402) generates a feedback signal including an instruction to apply the chest compressions one direction or another and/or closer to the ideal position.

According to various implementations, the flow monitor 402 can detect the presence of spontaneous circulation of the subject, in which the heart of the subject is spontaneously pumping a sufficient amount of blood through the body of the subject. In some cases, the flow monitor 402 detects a return of spontaneous circulation (ROSC). The flow monitor 402, for instance, can detect spontaneous circulation while the subject is receiving chest compressions. As noted previously, when the subject is receiving chest compressions, the chest compressions cause parallel or unidirectional blood flow in the artery 408 and the vein 410. In contrast, the pumping heart of the subject circulating blood through the subject's circulatory system causes antiparallel or bidirectional blood flow in the artery 408 and the vein 410. Thus, the flow monitor 402, in some cases, detects spontaneous circulation in response to detecting antiparallel or bidirectional blood flow in the artery 408 and vein 410.

In some cases, the flow monitor 402 detects spontaneous circulation using other techniques. In general, the heart is capable of pumping blood at a greater peak velocity than chest compressions. Therefore, the monitor 402 may detect spontaneous circulation by detecting that a blood velocity through the artery 408 or the vein 410 is greater than a threshold. Further, the blood velocity through the artery 408 or the vein 410 with respect to time during chest compressions is different than the blood velocity through the artery 408 or the vein 410 with respect to time during spontaneous circulation. According to some implementations, the flow monitor detects spontaneous circulation by determining that at least one frequency component of the volumetric flow rate over time through the artery 408 or vein 410 has changed. For instance, a shape or morphology of the volumetric flow rate over time can be indicative of spontaneous circulation.

Certain functionalities of the flow monitor 402 are enhanced by communications with other devices, such as the mechanical chest compression device 426 and a monitor-defibrillator 428. Collectively, any combination of the flow monitor 402, the mechanical chest compression device 426, and the monitor-defibrillator 428 may be a resuscitation system that is configured to resuscitate a single subject. For example, the flow monitor 402 is configured to modify and/or temporally gate measurements it performs based on communications from the mechanical chest compression device 426 and/or the monitor-defibrillator 428. In some cases, the flow monitor 402 receives a communication signal from the mechanical chest compression device 426 that indicates a frequency of chest compressions administered by the mechanical chest compression device 426, a timing of chest compressions administered by the mechanical chest compression device 426, or a time of a pause in the chest compressions administered by the mechanical chest compression device 426. In some cases, the flow monitor 402 is configured to remove a chest compression artifact from a blood velocity over time based on the communication signal from the mechanical chest compression device 426. For instance, the flow monitor 402 may remove an artifact from the blood velocity by applying a band reject filter centered around the chest compression frequency or by applying a comb filter that includes the chest compression frequency and one or more harmonics of the chest compression frequency. In some cases, the flow monitor 402 is configured to temporally gate a blood velocity measurement during a time window in which the mechanical chest compression device 426 has paused chest compressions or between chest compressions performed by the chest compression device 426. Using these techniques, in some cases, may enable the flow monitor 402 to more accurately identify spontaneous circulation of the subject by distinguishing between blood flow caused by the chest compressions and spontaneous blood flow induced by the heart of the subject.

In some cases, the monitor-defibrillator 428 transmits a communication signal to the flow monitor 402 indicating a time at which the monitor-defibrillator 428 is administering a treatment to the subject, such as an electrical shock or pace pulses. Because the electrical shock or pace pulses can interfere with the accuracy of other measurements performed by the flow monitor 402, the flow monitor 402 may gate the measurements at a time interval that omits the treatment to the subject. In some implementations, the flow monitor 402 includes sensitive electronics that could potentially be damaged when the treatment is administered to the subject. According to some examples, the flow monitor 402 includes a circuit with at least one switch that disconnects or otherwise shields the sensitive electronics during the treatment.

In some examples, the flow monitor 402 includes or is otherwise communicatively coupled to devices that include one or more sensors (not illustrated) configured to detect other physiological parameters. For example, the flow monitor 402 includes or is communicatively coupled with a sensor configured to detect an electrocardiogram (ECG), an oximetry sensor (e.g., a regional oximetry sensor, a pulse oximetry sensor, a cerebral oximetry sensor, or the like), or both.

In various implementations, the flow monitor 402 includes one or more accelerometers 430 configured to detect an acceleration of the flow monitor 402 and/or the skin 404 of the subject. The acceleration, for instance, is indicative of chest compressions administered to the subject or a pulse of the subject through the artery 408 or vein 410. Thus, in some cases, the flow monitor 402 detects whether the subject has a pulse based on the acceleration detected by the accelerometer(s) 430. At least one additional accelerometer 432 may further be disposed on another portion of the subject (e.g., the subject's chest) and may be configured to detect an acceleration indicative of the chest compressions. The flow monitor 402, in some implementations, removes a chest compression artifact of the acceleration detected by the accelerometer(s) 430 over time based on the acceleration detected by the additional accelerometer(s) 432 over time. In various implementations, the flow monitor 402 is configured to accurately detect spontaneous circulation or another condition of the subject based on the detected accelerations.

As used herein, the term "measurement" may refer to a blood velocity, a net blood flow volume, an ECG, an oxygenation of the subject's blood, an acceleration, or another physiological parameter of the subject. In some cases, the flow monitor 402 validates and/or temporally gates a first measurement in view of a second measurement. For example, if the ECG indicates that the heart of the subject is beating, but the blood velocity or net blood flow indicates that oxygenated blood is not circulating in the subject, then the flow monitor 402 may refrain from indicating that the subject has spontaneous circulation. In some cases, the flow monitor 402 may output a signal indicating that the subject has pulseless electrical activity (PEA).

In various cases, the flow monitor 402 is configured to detect blood flow in the brain of the subject. In some cases, the artery 408 is a part of the circle of Willis of the subject. If the subject is an adult, the skull of the subject may highly attenuate the first incident beam 414, the second incident beam 416, the first return beam 418, and the second return beam 420. For instance, the skull may have high attenuation with respect to ultrasound and/or light (e.g., at certain frequencies). Thus, in various examples, the flow monitor 402 is configured to direct the first incident beam 414, the second incident beam 416, the first return beam 418, and the second return beam 420 through a window in the skull. For instance, the flow monitor 402 is configured to monitor the artery 408 and/or vein 410 through a temple of the subject, an orbital socket of the subject, or an ear canal of the subject. In some cases, the flow monitor 402 is strapped to the head of the subject, such as in the form of an eyepatch.

In some cases, the operation of the flow monitor 402 is optimized in other ways to enable monitoring within the brain. For instance, if the first incident beam 414 and the second incident beam 416 include ultrasound, the ultrasound may have a frequency of less than 1 MHz. Although sub MHz ultrasound is incapable of generating high-resolution images, in some cases, the flow monitor 402 detects the flow through the artery 408 or vein 410 without generating an image of the artery 408 or vein 410. That is, sub MHz ultrasound is sufficient for Doppler flow detection in various implementations described herein.

Another way in which the flow monitor 402 is optimized for monitoring with the brain relates to the type of the artery 408 and/or vein 410 monitored by the flow monitor 402. Attenuation of an incident beam increases as it travels through an attenuating structure. Thus, in some cases, a distance between the flow monitor 402 (e.g., disposed on the skin 404 or eye) is less than a threshold distance.

In particular cases, it may be difficult to differentiate specific blood vessels in the brain (e.g., within the Circle of Willis) in which the first return beam 418 and/or the second return beam 420 are reflected and/or scattered. Further, it may be difficult to identify angles from which the blood vessels in the brain are disposed with respect to the flow monitor 402. Accordingly, it may be difficult to accurately quantify blood flow parameters of a blood vessel in the brain using techniques described herein. However, even detecting the presence or absence of movement of blood in the brain can provide helpful feedback in order to detect chest compression efficacy and/or spontaneous circulation. According to various implementations, the flow monitor 402 outputs an indication if the flow monitor 402 detects movement, or the absence of movement, or the relative difference of movement between the compression and decompression parts of CPR or the diastolic and systolic parts of a normal sinus rhythm of blood in the brain of the subject.

By detecting blood flow in the brain, the flow monitor 402 is configured to detect an efficacy of chest compressions being administered to the subject. Technologies that monitor blood flow in extremities can indirectly detect chest compression efficacy. However, because a primary purpose of chest compressions is to provide oxygenated blood to the brain of the subject, the flow monitor 402 is able to directly detect the efficacy of the chest compressions by monitoring blood flow in the brain. The flow monitor 402, for instance, can output a feedback signal (e.g., to the mechanical chest compression device 426 or to the user) indicating whether chest compressions are generating blood flow in the brain. In some cases, the feedback signal includes a metric indicative of the blood flow in the brain (e.g., a quantity that increases with an increase in a detected blood velocity or flow rate).

According to some examples, the receiver(s) 422 include one or more microphones configured to detect an audible sound 434 from the subject. For example, the flow monitor 402 may include the functionality of a sophisticated stethoscope. In some cases, the microphone(s) detect the audible sound 434 emitted by blood flowing through the artery 408 or vein 410. In some examples, the microphone(s) detect the audible sound 434 emitted by the heart of the subject. In various implementations, the audible sound 434 can be in an audible or inaudible range. The flow monitor 402, in various cases, detects the audible sound 434 at a sampling frequency, such as a sampling frequency of greater than 100 Hz, 1 kHz, or 10 kHz. In some implementations, the flow monitor 402 determines a condition of the subject based on the audible sound 434.

According to some cases, the output device(s) 424 include a speaker that outputs an audible signal indicative of the audible sound 434 detected by the flow monitor 402. For example, the output device(s) 424 output the sampled audible sound 434 into an audio headset of the user, or in an environment in which the user is present, at a volume that can be perceived by the user in an emergency scene. That is, the output device(s) 424 may amplify the audible sound 434. Some monitors output computer-generated sounds indicative of a heart rate of a subject, such as a "beep" sound whenever a QRS complex is detected in an ECG. According to some implementations, the audible sound 434 includes more diagnostic-relevant information that can be perceived by the user than a computerized "beep" sound. In some examples, a condition of the subject can be identified using the strength, velocity, or morphological characteristics of blood flow, but these features cannot necessarily be identified using audio output from a previous type of monitor. Furthermore, by outputting an amplified version of the audible sound 434 as an audible signal, the flow monitor 402 may convey potentially important information about the condition of the subject without visually outputting it on a display. The user, for example, may be monitoring other visual signals on the monitor-defibrillator 428, and thus the flow monitor 402 may enable cognitive offloading for the user.

In some implementations, the receiver(s) 422 include multiple microphones configured to detect audible sounds 434 from multiple locations on the subject's body. For instance, the microphones may detect the audible sound 434 from a right quadrant of the subject's chest and another audible sound 434 from a left quadrant of the subject's chest. The flow monitor 402, in some cases, analyzes the detected sounds for features indicative of a medical condition. For example, the flow monitor 402 detects the medical condition by detecting an anomaly in the detected sound. In some implementations, the flow monitor 402 selectively outputs a single one of the audible sounds based on an input signal received from the user. In some cases, the flow monitor 402 selectively outputs a single one of the audible sounds that is indicative of the medical condition. In various implementations, the output device(s) 424 are configured to output a signal indicating the medical condition.

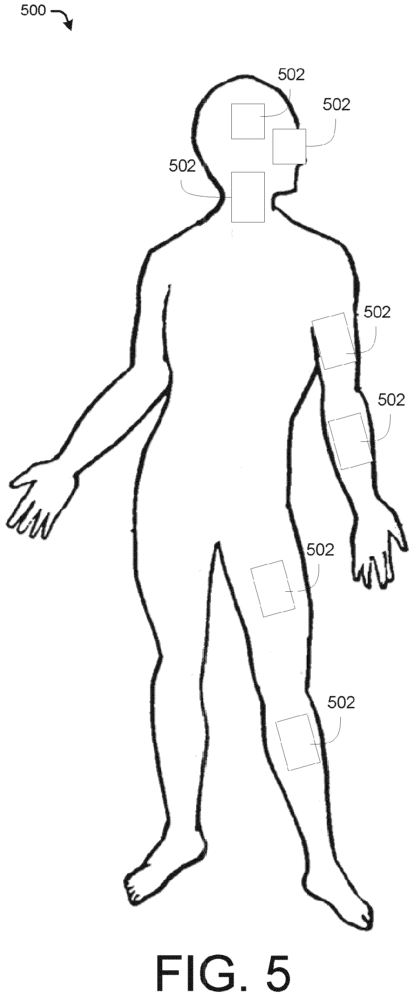

FIG. 5 illustrates various placements of the flow monitor 502 on the body of a subject 500. For instance, the flow monitor 502 may include at least one of the first sensor 106, the second sensor 108, the parameter analyzer 112, the flow monitor 304, or the flow monitor 402. The flow monitor 502 may be disposed on a temple, an orbital socket, an ear canal, a neck, an upper arm, a lower arm, an upper leg, or a lower leg of the subject 500. In some implementations, the flow monitor 502 includes multiple physical devices that are disposed on different parts of the body of the subject 500, simultaneously. In various implementations, the flow monitor 502 may be integrated into a cot or other support on which the subject 500 is disposed.

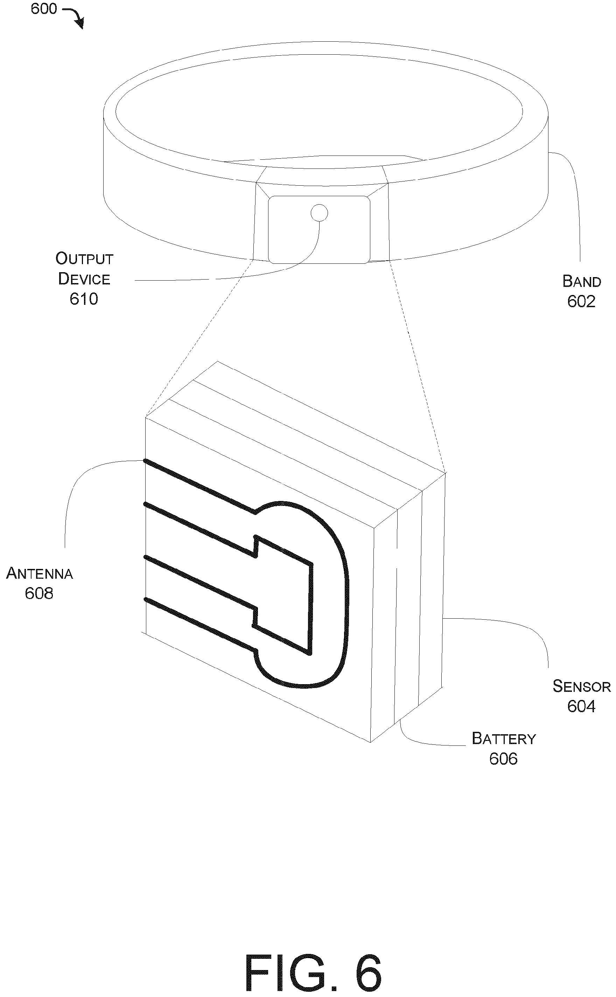

FIG. 6 illustrates the structure of an example flow monitor 600. The flow monitor 600, for instance, includes at least one of the first sensor 106, the second sensor 108, the parameter analyzer 112, the flow monitor 304, the flow monitor 402, or the flow monitor 502. In some cases, the flow monitor 600 is a patch that can be strapped to a subject or adhered to the skin of the subject. For instance, the flow monitor 600 includes a band 602 configured to be disposed around an appendage of the subject. According to some examples, the band 602 is configured to be disposed around a chest, abdomen, head, or neck of the subject.

In various implementations, the flow monitor 600 includes a sensor 604 configured to detect a physiological parameter indicative of blood flow through a subject. In some cases, the sensor 604 includes a microphone configured to detect a sound generated by the body of the subject. In some examples, the sensor 604 includes an ultrasound transducer configured to detect the velocity of blood flowing through at least one blood vessel of the subject and/or through the heart of the subject. In some examples, the sensor 604 includes an ultrasound transducer configured to detect heart wall motion. In some cases, the sensor 604 includes at least one transmitter and/or at least one receiver. In some examples, the sensor 604 includes one or more accelerometers.

The sensor 604, for instance, is powered by a battery 606. In some cases, the battery 606 is disposable. In some examples, the battery 606 is rechargeable.

The flow monitor 600, in various cases, further includes an antenna 608. The antenna 608, in various implementations, is configured to transmit and/or receive communication signals from an external device. In some cases, the antenna 608 enables the flow monitor 600 to communicate with another flow monitor or another type of medical device (e.g., an AED or monitor-defibrillator). For instance, the flow monitor 600 is configured to transmit, to an external device, an indication of the physiological parameter detected by the sensor 604. The antenna 608, in various cases, is powered by the battery 606.

According to various examples, the sensor 604, the battery 606, and the antenna 608 are arranged in respective layers of the flow monitor 600. For example, each layer may correspond to a circuit board (e.g., a PCB) that accommodates a respective component. In various cases, the layer corresponding to the sensor 604 is configured to be adjacent to the subject when the flow monitor 600 is disposed on the subject. The layer corresponding to the antenna 608, for example, faces away from the subject when the flow monitor 600 is disposed on the subject. Accordingly, the body of the subject may be prevented from interfering with wireless signals transmitted and/or received by the antenna 608.

In some examples, the flow monitor 600 further includes an output device 610. The output device 610 is configured to output a signal to a user. In some cases, the signal is based on the physiological parameter detected by the sensor 604. For instance, the output device 610 includes a light that is activated when a blood velocity detected by the sensor 604 remains below a threshold blood velocity for greater than a threshold period of time.

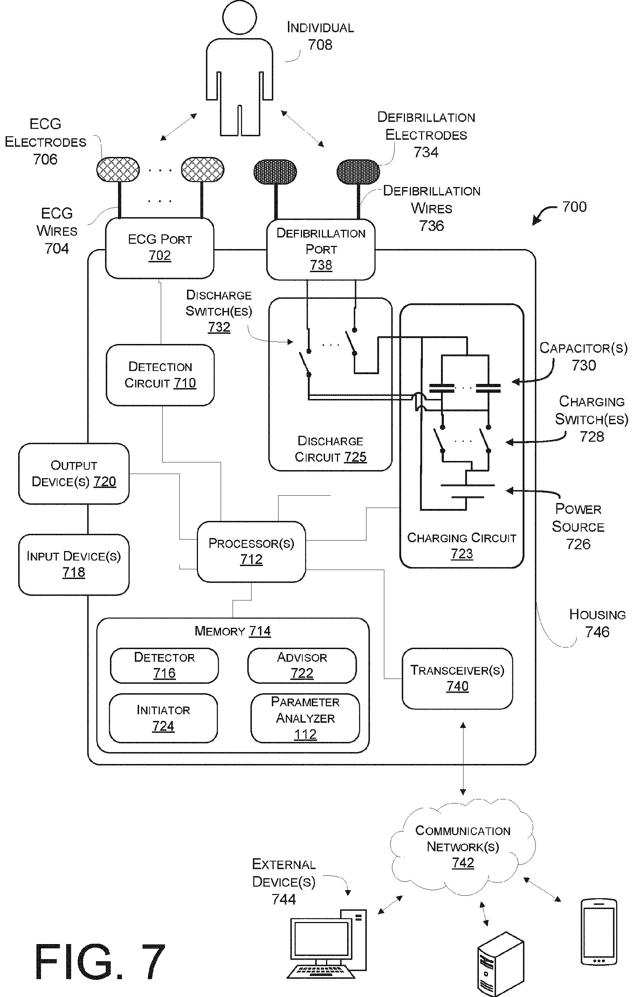

FIG. 7 illustrates an example of an external defibrillator 700 configured to perform various functions described herein. For example, the external defibrillator 700 is the monitor-defibrillator 428 described above with reference to FIG. 4.

The external defibrillator 700 includes an electrocardiogram (ECG) port 702 connected to multiple ECG wires 704. In some cases, the ECG wires 704 are removeable from the ECG port 702. For instance, the ECG wires 704 are plugged into the ECG port 702. The ECG wires 704 are connected to ECG electrodes 706, respectively. In various implementations, the ECG electrodes 706 are disposed on different locations on an individual 708. A detection circuit 710 is configured to detect relative voltages between the ECG electrodes 706. These voltages are indicative of the electrical activity of the heart of the individual 708.

In various implementations, the ECG electrodes 706 are in contact with the different locations on the skin of the individual 708. In some examples, a first one of the ECG electrodes 706 is placed on the skin between the heart and right arm of the individual 708, a second one of the ECG electrodes 706 is placed on the skin between the heart and left arm of the individual 708, and a third one of the ECG electrodes 706 is placed on the skin between the heart and a leg (either the left leg or the right leg) of the individual 708. In these examples, the detection circuit 710 is configured to measure the relative voltages between the first, second, and third ECG electrodes 706. Respective pairings of the ECG electrodes 706 are referred to as "leads," and the voltages between the pairs of ECG electrodes 706 are known as "lead voltages." In some examples, more than three ECG electrodes 706 are included, such that 5-lead or 12-lead ECG signals are detected by the detection circuit 710.

The detection circuit 710 includes at least one analog circuit, at least one digital circuit, or a combination thereof. The detection circuit 710 receives the analog electrical signals from the ECG electrodes 706, via the ECG port 702 and the ECG wires 704. In some cases, the detection circuit 710 includes one or more analog filters configured to filter noise and/or artifact from the electrical signals. The detection circuit 710 includes an analog-to-digital converter (ADC) in various examples. The detection circuit 710 generates a digital signal indicative of the analog electrical signals from the ECG electrodes 706. This digital signal can be referred to as an "ECG signal" or an "ECG."

In some cases, the detection circuit 710 further detects an electrical impedance between at least one pair of the ECG electrodes 706. For example, the detection circuit 710 includes, or otherwise controls, a power source that applies a known voltage (or current) across a pair of the ECG electrodes 706 and detects a resultant current (or voltage) between the pair of the ECG electrodes 706. The impedance is generated based on the applied signal (voltage or current) and the resultant signal (current or voltage). In various cases, the impedance corresponds to respiration of the individual 708, chest compressions performed on the individual 708, and other physiological states of the individual 708. In various examples, the detection circuit 710 includes one or more analog filters configured to filter noise and/or artifact from the resultant signal. The detection circuit 710 generates a digital signal indicative of the impedance using an ADC. This digital signal can be referred to as an "impedance signal" or an "impedance."

The detection circuit 710 provides the ECG signal and/or the impedance signal one or more processors 712 in the external defibrillator 700. In some implementations, the processor(s) 712 includes a central processing unit (CPU), a graphics processing unit (GPU), digital signal processing unit (DPU), other processing unit or component known in the art, or any combination thereof.

The processor(s) 712 is operably connected to memory 714. In various implementations, the memory 714 is volatile (such as random access memory (RAM)), non-volatile (such as read only memory (ROM), flash memory, etc.) or some combination of the two. The memory 714 stores instructions that, when executed by the processor(s) 712, causes the processor(s) 712 to perform various operations. In various examples, the memory 714 stores methods, threads, processes, applications, objects, modules, any other sort of executable instruction, or a combination thereof. In some cases, the memory 714 stores files, databases, or a combination thereof. In some examples, the memory 714 includes, but is not limited to, RAM, ROM, electrically erasable programmable read-only memory (EEPROM), flash memory, or any other memory technology. In some examples, the memory 714 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 712 and/or the external defibrillator 700. In some cases, the memory 714 at least temporarily stores the ECG signal and/or the impedance signal.

In various examples, the memory 714 includes a detector 716, which causes the processor(s) 712 to determine, based on the ECG signal and/or the impedance signal, whether the individual 708 is exhibiting a particular heart rhythm. For instance, the processor(s) 712 determines whether the individual 708 is experiencing a shockable rhythm that is treatable by defibrillation. Examples of shockable rhythms include ventricular fibrillation (VF) and ventricular tachycardia (VT). In some examples, the processor(s) 712 determines whether any of a variety of different rhythms (e.g., asystole, sinus rhythm, atrial fibrillation (AF), etc.) are present in the ECG signal.

The processor(s) 712 is operably connected to one or more input devices 718 and one or more output devices 720. Collectively, the input device(s) 718 and the output device(s) 720 function as an interface between a user and the defibrillator 700. The input device(s) 718 is configured to receive an input from a user and includes at least one of a switch, a keypad, a cursor control, a touch-sensitive display, a voice input device (e.g., a microphone), a haptic feedback device (e.g., a gyroscope), or any combination thereof. The output device(s) 720 includes at least one of a display, a speaker, a haptic output device, a printer, a light indicator such as an LED, or any combination thereof. In various examples, the processor(s) 712 causes a display among the input device(s) 718 to visually output a waveform of the ECG signal and/or the impedance signal. In some implementations, the input device(s) 718 includes one or more touch sensors, the output device(s) 720 includes a display screen, and the touch sensor(s) are integrated with the display screen. Thus, in some cases, the external defibrillator 700 includes a touchscreen configured to receive user input signal(s) and visually output physiological parameters, such as the ECG signal and/or the impedance signal.

In some examples, the memory 714 includes an advisor 722, which, when executed by the processor(s) 712, causes the processor(s) 712 to generate advice and/or control the output device(s) 720 to output the advice to a user (e.g., a rescuer). In some examples, the processor(s) 712 provides, or causes the output device(s) 720 to provide, an instruction to perform CPR on the individual 708. In some cases, the processor(s) 712 evaluates, based on the ECG signal, the impedance signal, or other physiological parameters, CPR being performed on the individual 708 and causes the output device(s) 720 to provide feedback about the CPR in the instruction. According to some examples, the processor(s) 712, upon identifying that a shockable rhythm is present in the ECG signal, causes the output device(s) 720 to output an instruction and/or recommendation to administer a defibrillation shock to the individual 708.

The memory 714 also includes an initiator 724 which is configured to, when executed by the processor(s) 712, cause the processor(s) 712 to control other elements of the external defibrillator 700 in order to administer a defibrillation shock to the individual 708. In some examples, the processor(s) 712 executing the discharge circuit 725 is configured to selectively cause the administration of the defibrillation shock based on determining that the individual 708 is exhibiting the shockable rhythm and/or based on an input from a user (received, e.g., by the input device(s) 718. In some cases, the processor(s) 712 causes the defibrillation shock to be output at a particular time, which is determined by the processor(s) 712 based on the ECG signal and/or the impedance signal.

In some examples, the memory 714 includes instructions for performing one or more functions of the parameter analyzer 112. For instance, the instructions, when executed by the processor(s) 712, cause the processor(s) 712 to execute functions of the parameter analyzer 112 described herein.

The processor(s) 712 is operably connected to a charging circuit 723 and a discharge circuit 725. In various implementations, the charging circuit 723 includes a power source 726, one or more charging switches 728, and one or more capacitors 730. The power source 726 includes, for instance, a battery. The processor(s) 712 initiates a defibrillation shock by causing the power source 726 to charge at least one capacitor among the capacitor(s) 730. For example, the processor(s) 712 activates at least one of the charging switch(es) 728 in the charging circuit 723 to complete a first circuit connecting the power source 726 and the capacitor to be charged. Then, the processor(s) 712 causes the discharge circuit 725 to discharge energy stored in the charged capacitor across a pair of defibrillation electrodes 734, which are in contact with the individual 708. For example, the processor(s) 712 deactivates the charging switch(es) 728 completing the first circuit between the capacitor(s) 730 and the power source 726, and activates one or more discharge switches 732 completing a second circuit connecting the charged capacitor 730 and at least a portion of the individual 708 disposed between defibrillation electrodes 734.

The energy is discharged from the defibrillation electrodes 734 in the form of a defibrillation shock. For example, the defibrillation electrodes 734 are connected to the skin of the individual 708 and located at positions on different sides of the heart of the individual 708, such that the defibrillation shock is applied across the heart of the individual 708. The defibrillation shock, in various examples, depolarizes a significant number of heart cells in a short amount of time. The defibrillation shock, for example, interrupts the propagation of the shockable rhythm (e.g., VF or VT) through the heart. In some examples, the defibrillation shock is 200 J or greater with a duration of about 0.015 seconds. In some cases, the defibrillation shock has a multiphasic (e.g., biphasic) waveform. The discharge switch(es) 732 are controlled by the processor(s) 712, for example. In various implementations, the defibrillation electrodes 734 are connected to defibrillation wires 736. The defibrillation wires 736 are connected to a defibrillation port 738, in implementations. According to various examples, the defibrillation wires 736 are removable from the defibrillation port 738. For example, the defibrillation wires 736 are plugged into the defibrillation port 738.

In various implementations, the processor(s) 712 is operably connected to one or more transceivers 740 that transmit and/or receive data over one or more communication networks 742. For example, the transceiver(s) 740 includes a network interface card (NIC), a network adapter, a local area network (LAN) adapter, or a physical, virtual, or logical address to connect to the various external devices and/or systems. In various examples, the transceiver(s) 740 includes any sort of wireless transceivers capable of engaging in wireless communication (e.g., radio frequency (RF) communication). For example, the communication network(s) 742 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 740 includes other wireless modems, such as a modem for engaging in WI-FI^{®}, WIGIG^{®}, WIMAX^{®}, BLUETOOTH^{®}, or infrared communication over the communication network(s) 742.

The defibrillator 700 is configured to transmit and/or receive data (e.g., ECG data, impedance data, data indicative of one or more detected heart rhythms of the individual 708, data indicative of one or more defibrillation shocks administered to the individual 708, etc.) with one or more external devices 744 via the communication network(s) 742. The external devices 744 include, for instance, mobile devices (e.g., mobile phones, smart watches, etc.), Internet of Things (IoT) devices, medical devices (e.g., a flow monitor), computers (e.g., laptop devices, servers, etc.), or any other type of computing device configured to communicate over the communication network(s) 742. In some examples, the external device(s) 744 is located remotely from the defibrillator 700, such as at a remote clinical environment (e.g., a hospital). According to various implementations, the processor(s) 712 causes the transceiver(s) 740 to transmit data to the external device(s) 744. In some cases, the transceiver(s) 740 receives data from the external device(s) 744 and the transceiver(s) 740 provide the received data to the processor(s) 712 for further analysis.

In various implementations, the external defibrillator 700 also includes a housing 746 that at least partially encloses other elements of the external defibrillator 700. For example, the housing 746 encloses the detection circuit 710, the processor(s) 712, the memory 714, the charging circuit 723, the transceiver(s) 740, or any combination thereof. In some cases, the input device(s) 718 and output device(s) 720 extend from an interior space at least partially surrounded by the housing 746 through a wall of the housing 746. In various examples, the housing 746 acts as a barrier to moisture, electrical interference, and/or dust, thereby protecting various components in the external defibrillator 700 from damage.

In some implementations, the external defibrillator 700 is an automated external defibrillator (AED) operated by an untrained user (e.g., a bystander, layperson, etc.) and can be operated in an automatic mode. In automatic mode, the processor(s) 712 automatically identifies a rhythm in the ECG signal, makes a decision whether to administer a defibrillation shock, charges the capacitor(s) 730, discharges the capacitor(s) 730, or any combination thereof. In some cases, the processor(s) 712 controls the output device(s) 720 to output (e.g., display) a simplified user interface to the untrained user. For example, the processor(s) 712 refrains from causing the output device(s) 720 to display a waveform of the ECG signal and/or the impedance signal to the untrained user, in order to simplify operation of the external defibrillator 700.

In some examples, the external defibrillator 700 is a monitor-defibrillator utilized by a trained user (e.g., a clinician, an emergency responder, etc.) and can be operated in a manual mode or the automatic mode. When the external defibrillator 700 operates in manual mode, the processor(s) 712 cause the output device(s) 720 to display a variety of information that may be relevant to the trained user, such as waveforms indicating the ECG data and/or impedance data, notifications about detected heart rhythms, and the like.

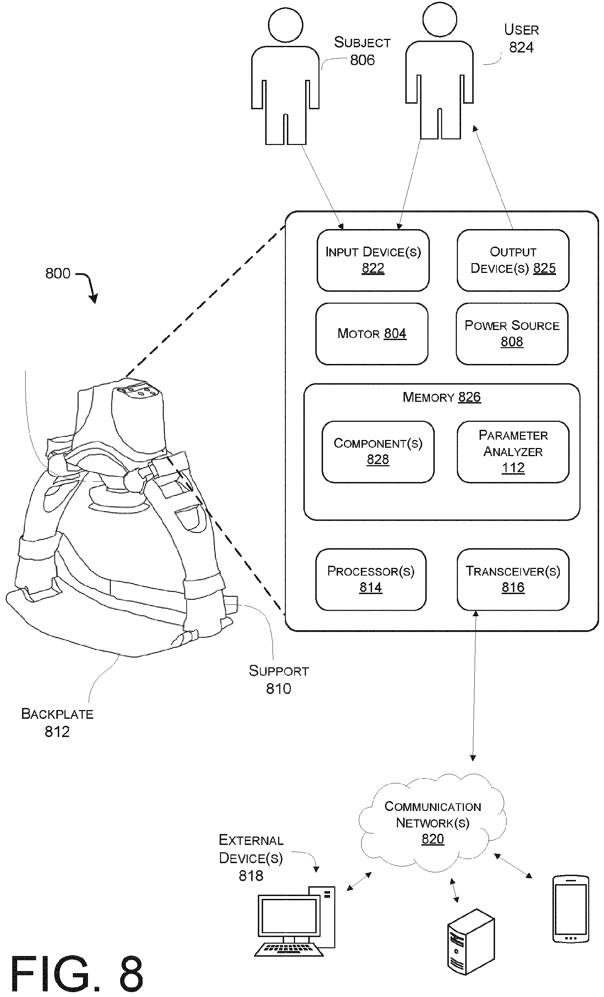

FIG. 8 illustrates a chest compression device 800 configured to perform various functions described herein. For example, the chest compression device 800 is the mechanical chest compression device 426 described with reference to FIG. 4.

In various implementations, the chest compression device 800 includes a compressor 802 that is operatively coupled to a motor 804. The compressor 802 is configured to physically administer a force to the chest of a subject 806 that compresses the chest of the subject 806. In some examples, the compressor 802 includes at least one piston that periodically moves between two positions (e.g., a compressed position and a release position) at a compression frequency. For example, when the piston is positioned on the chest of the subject 806, the piston compresses the chest when the piston is moved into the compressed position. A suction cup may be positioned on a tip of the piston, such that the suction cup contacts the chest of the subject 806 during operation. In various cases, the compressor 802 includes a band that periodically tightens to a first tension and loosens to a second tension at a compression frequency. For instance, when the band is disposed around the chest of the subject 806, the band compresses the chest when the band tightens.

The motor 804 is configured to convert electrical energy stored in a power source 808 into mechanical energy that moves and/or tightens the compressor 802, thereby causing the compressor 802 to administer the force to the chest of the subject 806. In various implementations, the power source 808 is portable. For instance, the power source 808 includes at least one rechargeable (e.g., lithium-ion) battery. In some cases, the power source 808 supplies electrical energy to one or more elements of the chest compression device 800 described herein.

In various cases, the chest compression device 800 includes a support 810 that is physically coupled to the compressor 802, such that the compressor 802 maintains a position relative to the subject 806 during operation. In some implementations, the support 810 is physically coupled to a backplate 812, cot, or other external structure with a fixed position relative to the subject 806. According to some cases, the support 810 is physically coupled to a portion of the subject 806, such as wrists of the subject 806.

The operation of the chest compression device 800 may be controlled by at least one processor 814. In various implementations, the motor 804 is communicatively coupled to the processor(s) 814. Specifically, the processor(s) 814 is configured to output a control signal to the motor 804 that causes the motor 804 to actuate or otherwise adjust the compressor 802. For instance, the motor 804 causes the compressor 802 to administer the compressions to the subject 806 based on the control signal. In some cases, the control signal indicates one or more treatment parameters of the compressions. Examples of treatment parameters include a frequency, timing, depth, force, position, velocity, and acceleration of the compressor 802 administering the compressions. According to various cases, the control signal causes the motor 804 to cease compressions, such as after ROSC has been detected.

In various implementations, the chest compression device 800 includes at least one transceiver 816 configured to communicate with at least one external device 818 over one or more communication networks 820. Any wired and/or wireless communication network described herein can be included in the communication network(s) 820 illustrated in FIG. 8. The external device(s) 818, for example, includes at least one of a flow monitor, a monitor-defibrillator, an AED, an ECMO device, a ventilation device, a subject monitor, a mobile phone, a server, or a computing device. In some implementations, the transceiver(s) 816 is configured to communicate with the external device(s) 818 by transmitting and/or receiving signals wirelessly. For example, the transceiver(s) 816 includes a NIC, a network adapter, a LAN adapter, or a physical, virtual, or logical address to connect to the various external devices and/or systems. In various examples, the transceiver(s) 816 includes any sort of wireless transceivers capable of engaging in wireless communication (e.g., RF communication). For example, the communication network(s) 820 includes one or more wireless networks that include a 3GPP network, such as an LTE RAN (e.g., over one or more LTE bands), an NR RAN (e.g., over one or more NR bands), or a combination thereof. In some cases, the transceiver(s) 816 includes other wireless modems, such as a modem for engaging in WI-FI^{®}, WIGIG^{®}, WIMAX^{®}, BLUETOOTH^{®}, or infrared communication over the communication network(s) 820. The signals, in various cases, encode data in the form of data packets, datagrams, or the like. In some cases, the signals are transmitted as compressions are being administered by the chest compression device 800 (e.g., for real-time feedback by the external device(s) 818), after compressions are administered by the chest compression device 800 (e.g., for post-event review at the external device 818), or a combination thereof.

In various cases, the processor(s) 814 generates the control signal based on data encoded in the signals received from the external device(s) 818. For instance, the signals include an instruction to initiate the compressions, and the processor(s) 814 instructs the motor 804 to begin actuating the compressor 802 in accordance with the signals.

In some cases, the chest compression device 800 includes at least one input device 822. In various examples, the input device(s) 822 is configured to receive an input signal from a user 824, who may be a rescuer treating the subject 806. Examples of the input device(s) 822 include, for instance, a switch, a keypad, a cursor control, a touch-sensitive display, a voice input device (e.g., a microphone), a haptic feedback device (e.g., a gyroscope), or any combination thereof. In various implementations, the processor(s) 814 generate the control signal based on the input signal. For instance, the processor(s) 814 generate the control signal to adjust a frequency of the compressions based on the chest compression device 800 detecting a selection by the user 824 of a user interface element displayed on a touchscreen or detecting the user 824 pressing a button integrated with an external housing of the chest compression device 800.

According to some examples, the input device(s) 822 include one or more sensors. The sensor(s), for example, is configured to detect a physiological parameter of the subject 806. In some implementations, the sensor(s) is configured to detect a state parameter of the chest compression device 800, such as a position of the compressor 802 with respect to the subject 806 or the backplate 812, a force administered by the compressor 802 on the subject 806, a force administered onto the backplate 812 by the body of the subject 806 during a compression, or the like. According to some implementations, the signals transmitted by the transceiver(s) 816 indicate the physiological parameter(s) and/or the state parameter(s).

The chest compression device 800 further includes at least one output device 825, in various implementations. Examples of the output device(s) 825 include, for instance, least one of a display (e.g., a projector, an LED screen, etc.), a speaker, a haptic output device, a printer, a light source such as an LED, or any combination thereof. In some implementations, the output device(s) 825 include a screen configured to display various parameters detected by and/or reported to the chest compression device 800, a charge level of the power source 808, a timer indicating a time since compressions were initiated or paused, and other relevant information.

The chest compression device 800 further includes memory 826. In various implementations, the memory 826 is volatile (such as RAM), non-volatile (such as ROM, flash memory, etc.) or some combination of the two. The memory 826 stores instructions that, when executed by the processor(s) 814, causes the processor(s) 814 to perform various operations. In various examples, the memory 826 stores methods, threads, processes, applications, objects, modules, any other sort of executable instruction, or a combination thereof. In some cases, the memory 826 stores files, databases, or a combination thereof. In some examples, the memory 826 includes, but is not limited to, RAM, ROM, 8PROM, flash memory, or any other memory technology. In some examples, the memory 826 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 826 stores instructions, programs, threads, objects, data, or any combination thereof, that cause the processor(s) 814 to perform various functions. In various cases, the memory 826 stores one or more parameters that are detected by the chest compression device 800 and/or reported to the chest compression device 800.

In implementations of the present disclosure, the memory 826 also stores one or more components 828. The component(s) 828 include programs, instructions, files, databases, models, or any other type of data that causes the device 800 to perform any of the functions described herein. In various cases, the memory 826 also stores instructions for performing one or more functions of the parameter analyzer 112.

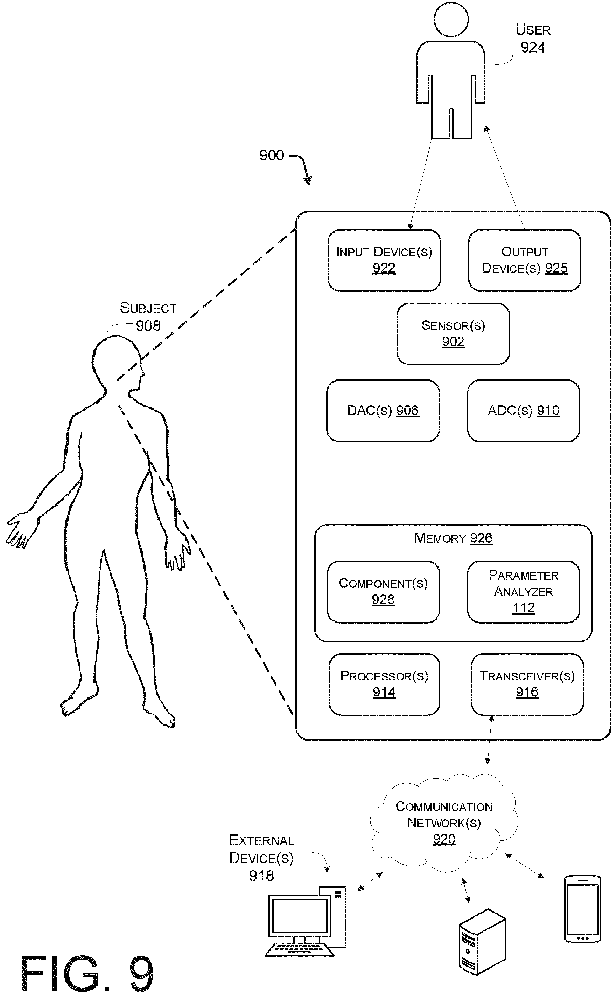

FIG. 9 illustrates a flow monitor 900 configured to perform various functions described herein. For example, the flow monitor 900 includes at least one of the first sensor 106, the second sensor 108, the parameter analyzer 112, the flow monitor 304, the flow monitor 402, the flow monitor 502, or the flow monitor 600.

In various implementations, the flow monitor 900 includes one or more transmitters 902, one or more receivers 904, one or more DACs 906, and one or more digital to analog converters 910. The transmitter(s) 902 is configured to emit an incident beam into a subject 908. The incident beam includes infrared light and/or ultrasound. For instance, the transmitter(s) 902 include one or more light sources (e.g., one or more LEDs) or one or more piezoelectric crystals, MEMS transducers, or other electro-mechanical conversion device or material. In some examples, the flow monitor 900 is positioned such that the incident beam is transmitted through a window in the skull of the subject 908, such as a temple, an orbital cavity, a nasal cavity, or an ear canal. In some cases, the transmitter(s) 902 emits one or more incident beams toward an artery supplying blood to a portion of the body of the subject 908 and a vein transporting blood out of the portion of the body of the subject 908.

In various implementations, the incident beam is reflected or otherwise scattered by blood in a blood vessel of the subject 908. The receiver(s) 904 is configured to detect within a return beam that includes signals from the reflection or scatter from the blood in the blood vessel. For example, the receiver(s) 904 include one or more light sensors or one or more piezoelectric crystals, MEMS transducers, or other electro-mechanical conversion device or material. In various cases, the flow monitor 900 includes one or more transceivers that embody the transmitter(s) 902 and receiver(s) 904.

The operation of the flow monitor 900 may be controlled by at least one processor 914. The processor(s) 914 are communicatively coupled to the DAC(s) 906 and the ADC(s) 910. The DAC(s) 906 is configured to convert digital signals output by the processor(s) 914 into analog signals, such as analog signals that induce the transmitter(s) 902 to emit the incident beam. The ADC(S) 910 is configured to convert analog signals generated by the receiver(s) 904 (e.g., induced by detecting the return beam) into digital signals that are received by the processor(s) 914. The processor(s) 914 is configured to control the transmitter(s) 902 and to analyze the signals detected by the receiver(s) 904. The processor(s) 914, for instance, analyze the digital signals from the ADC(s) 910, which are indicative of the return beam detected by the receiver(s) 904, in order to determine a flow velocity of the blood through the blood vessel. In some examples, the flow monitor 900 includes one or more switches (e.g., MOSFETs) connected to an array of transmitters within the transmitter(s) 902 that respectively connect the transmitter(s) 902 between one or more power rails (e.g., negative and/or positive power rails) and ground. The processor(s) 914, for instance, cause the switch(es) to connect each transmitter among the transmitter(s) 902 between ground and the power rail(s) in a periodic waveform, which causes the transmitter(s) 902 to selectively output incident beam(s) in accordance with the periodic waveform. In some cases, the waveform has a three half-cycle waveform and goes from a positive voltage, to a negative voltage, to a positive voltage, then to ground. In some cases, more than one positive power rail or more than one negative power rail is used for the same waveform.

In various implementations, the flow monitor 900 includes at least one transceiver 916 configured to communicate with at least one external device 918 over one or more communication networks 920. Any communication network described herein can be included in the communication network(s) 920 illustrated in FIG. 9. The external device(s) 918, for example, includes at least one of a mechanical chest compression device, a monitor-defibrillator, an AED, an ECMO device, a ventilation device, a subject monitor, a mobile phone, a server, or a computing device. In some implementations, the transceiver(s) 916 is configured to communicate with the external device(s) 918 by transmitting and/or receiving signals in a wired fashion and/or wirelessly. For example, the transceiver(s) 916 includes a NIC, a network adapter, a LAN adapter, or a physical, virtual, or logical address to connect to the various external devices and/or systems. In various examples, the transceiver(s) 916 includes any sort of wireless transceivers capable of engaging in wireless communication (e.g., RF communication). For example, the communication network(s) 920 includes one or more wireless networks that include a 3GPP network, such as an LTE RAN (e.g., over one or more LTE bands), an NR RAN (e.g., over one or more NR bands), or a combination thereof. In some cases, the transceiver(s) 916 includes other wireless modems, such as a modem for engaging in WI-FI^{®}, WIGIG^{®}, WIMAX^{®}, BLUETOOTH^{®}, or infrared communication over the communication network(s) 920. The signals, in various cases, encode data in the form of data packets, datagrams, or the like. In some cases, the signals are transmitted as compressions are being administered by the flow monitor 900 (e.g., for real-time feedback by the external device(s) 918), after compressions are administered by the flow monitor 900 (e.g., for post-event review at the external device 918), or a combination thereof.

In various cases, the processor(s) 914 generates the control signal based on data encoded in the signals received from the external device(s) 918. For instance, the signals include an instruction to initiate monitoring, and the processor(s) 914 outputs a control signal that causes the transmitter(s) 902 to emit the incident beam.

In some cases, the flow monitor 900 includes at least one input device 922. In various examples, the input device(s) 922 is configured to receive an input signal from a user 924, who may be a rescuer treating the subject 908. Examples of the input device(s) 922 include, for instance, a a keypad, a cursor control, a touch-sensitive display, a voice input device (e.g., a microphone), a haptic feedback device (e.g., a gyroscope), or any combination thereof. In various implementations, the processor(s) 914 generate the control signal based on the input signal. For instance, the processor(s) 914 generate the control signal to adjust a frequency of the compressions based on the flow monitor 900 detecting a selection by the user 924 of a user interface element displayed on a touchscreen or detecting the user 924 pressing a button integrated with an external housing of the flow monitor 900.

According to some examples, the input device(s) 922 include one or more sensors. The sensor(s), for example, is configured to detect a physiological parameter of the subject 908. In some cases, the sensor(s) include one or more microphones, one or more accelerometers, one or more oximetry sensors, or one or more ECG sensors. The sensor(s), for instance, is configured to detect one or more physiological parameters. In some implementations, the sensor(s) is configured to detect a state parameter of the flow monitor 900, such as a position of the flow monitor 900 with respect to the subject 908 or the like. According to some implementations, the signals transmitted by the transceiver(s) 916 indicate the physiological parameter(s) and/or the state parameter(s).

The flow monitor 900 further includes at least one output device 925, in various implementations. Examples of the output device(s) 925 include, for instance, least one of a display (e.g., a projector, an LED screen, etc.), a speaker, a haptic output device, a printer, a light such as an LED, or any combination thereof. In some implementations, the output device(s) 925 include a screen configured to display various parameters detected by and/or reported to the flow monitor 900, a battery level of the flow monitor 900, and other relevant information.

The flow monitor 900 further includes memory 926. In various implementations, the memory 926 is volatile (such as RAM), non-volatile (such as ROM, flash memory, etc.) or some combination of the two. The memory 926 stores instructions that, when executed by the processor(s) 914, causes the processor(s) 914 to perform various operations. In various examples, the memory 926 stores methods, threads, processes, applications, objects, modules, any other sort of executable instruction, or a combination thereof. In some cases, the memory 926 stores files, databases, or a combination thereof. In some examples, the memory 926 includes, but is not limited to, RAM, ROM, 8PROM, flash memory, or any other memory technology. In some examples, the memory 926 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 926 stores instructions, programs, threads, objects, data, or any combination thereof, that cause the processor(s) 914 to perform various functions. In various cases, the memory 926 stores one or more parameters that are detected by the flow monitor 900 and/or reported to the flow monitor 900.

In implementations of the present disclosure, the memory 926 also stores one or more components 928. The component(s) 928 include programs, instructions, files, databases, models, or any other type of data that causes the flow monitor 900 to perform any of the functions described herein. For instance, the memory 926 stores instructions for executing one or more of the functions of the parameter analyzer 112.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. Each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein.

## Claims

1. A medical device, comprising:
a first sensor (106) configured to detect a first physiological parameter of a subject (104);
a second sensor (108,304) configured to detect a second physiological parameter of the subject,
an output device (114]; and
a processor configured to:
determine, by analyzing the first physiological parameter, that the subject is receiving a treatment during a first time interval;
in response to determining that the subject is receiving the treatment during the first time interval, determine, by analyzing the second physiological parameter detected during a second time interval that is different than the first time interval, a condition of the subject; and
cause the output device to output an indication of the condition;
wherein the second physiological parameter includes a blood flow rate, a net flow volume, or a volumetric flow rate,
wherein the medical device further includes a third sensor (308) configured to detect a blood
pressure of the subject, and
wherein the processor is configured to analyze the second physiological parameter and the blood pressure of the subject to determine the condition of the subject.

2. The medical device of claim 1, wherein the treatment comprises a chest compression, a pace pulse, assisted ventilation, or an electrical shock, and
wherein the condition comprises a pulse, pulseless electrical activity (PEA), a seizure, a spontaneous breath, or an arrhythmia.

3. The medical device of any of claims 1-2, wherein the processor is configured to determine, by analyzing the second physiological parameter detected during the second time interval that is different than the first time interval, the condition of the subject by:
identifying, in the second physiological parameter, an artifact indicative of the condition.

4. The medical device of any of claims 1-3, wherein the output device is configured to output the indication of the condition by transmitting, to a mechanical chest compression device, an instruction to cease applying chest compressions to the subject.

5. The medical device of any of claims 1-4, wherein the output device is configured to display a waveform indicative of the second physiological parameter, the waveform comprising a first portion corresponding to the first time interval and a second portion corresponding to the second time interval,
wherein the first portion comprises a different color or style than the second portion.

6. The medical device of any of claims 1-5, wherein:
the first sensor comprises an accelerometer configured to detect an acceleration of a chest of a subject over the first time interval and the second time interval;
the second sensor comprises a pulse sensor configured to detect a parameter indicative of blood flow through a blood vessel of the subject over the first time interval and the second time interval;
the processor is configured to:
detect, by analyzing the acceleration of the chest of the subject, a first chest compression performed on the subject during the first time interval;
detect, by analyzing the acceleration of the chest of the subject, an absence of a second chest compression performed on the subject during the second time interval;
in response to detecting the absence of the second chest compression performed on the subject during the second time interval:
detect, by analyzing the parameter indicative of blood flow through the blood vessel of the subject, a pulse of the subject during the second time interval; and
cause the output device to output a signal indicating the pulse.

7. The medical device of claim 6, wherein the processor is configured to detect the pulse of the subject during the second time interval by:
calculating a derivative of the blood flow during the second time interval with respect to time; and
determining that the derivative decreases and crosses zero.

8. The medical device of claim 6 or 7, wherein the pulse sensor comprises:
an emitter configured to output an incident beam; and
a receiver configured to detect the parameter indicative of blood flow through the blood vessel of the subject by detecting a reflection or scatter of the incident beam from the blood flow through the blood vessel of the subject, and
wherein the incident beam comprises ultrasound or infrared light.

9. A method, comprising:
identifying a first physiological parameter of a subject detected by a first sensor;
identifying a second physiological parameter of the subject detected by a second sensor,
wherein the second physiological parameter includes a blood flow rate, a net flow volume, or a volumetric flow rate;
detecting, by a third sensor, a blood pressure of the subject;
determining, by analyzing the first physiological parameter, that the subject is receiving a treatment during a first time interval;
in response to determining that the subject is receiving the treatment during the first time interval, determining, by analyzing the second physiological parameter detected during a second time interval that is different than the first time interval, a condition of the subject; and
outputting an indication of the condition,
wherein the condition of the subject is determined by analyzing the second physiological parameter and the blood pressure of the subject.

10. The method of claim 9, wherein the second physiological parameter comprises a volume of a blood vessel of the subject,
wherein determining the second physiological parameter of the subject detected by a second sensor comprises determining the second physiological parameter during a first instance of a cyclical event and during a second instance of the cyclical event,
wherein the method further comprises:
detecting a blood pressure of the blood vessel of the subject during the first instance of a cyclical event and during the second instance of the cyclical event, the first instance and the second instance occurring during the second time interval;
determining a slope of a first pressure-volume (PV) loop corresponding to the blood pressure and the second physiological parameter during the first instance of the cyclical event;
determining a slope of a second PV loop corresponding to the blood pressure and the second physiological parameter during the second instance of the cyclical event; and
determining a difference between the slope of the first PV loop and the slope of the second PV loop,
wherein determining the condition of the subject comprises determining that the difference between the slope of the first PV loop and the slope of the second PV loop is less than a first threshold or greater than a second threshold, and
wherein the cyclical event comprises a cardiac cycle, a chest compression cycle, or a cardiopulmonary resuscitation (CPR) cycle.

11. The method of any of claims 9 or 10, wherein the treatment comprises a chest compression, a pace pulse, assisted ventilation, or an electrical shock, and wherein the condition comprises a pulse, pulseless electrical activity (PEA), a seizure, a spontaneous breath, administration of a medication, or an arrhythmia; and/or
wherein determining, by analyzing the second physiological parameter detected during the second time interval that is different than the first time interval, the condition of the subject comprises detecting, in the second physiological parameter, an artifact indicative of the condition.

12. The method of any of claims 9-11, wherein
outputting the indication of the condition comprises transmitting, to a mechanical chest compression device, an instruction to cease applying chest compressions to the subject; and/or
outputting the indication of the condition comprises outputting, to a user, an instruction to administer a second treatment to the subject; and/or
wherein outputting the indication of the condition comprises:
outputting the indication that the condition is stable, worsening, or improving.

13. The method of any of claims 9-12, further comprising:
displaying a waveform indicative of the second physiological parameter, the waveform comprising a first portion corresponding to the first time interval and a second portion corresponding to the second time interval,
wherein the first portion comprises a different color or style than the second portion.

## Patentansprüche

1. Medizinische Vorrichtung, umfassend:
einen ersten Sensor (106), der dazu konfiguriert ist, einen ersten physiologischen Parameter eines Subjekts (104) zu erfassen;
einen zweiten Sensor (108, 304), der dazu konfiguriert ist, einen zweiten physiologischen Parameter des Subjekts zu erfassen;
eine Ausgabevorrichtung (114); und
einen Prozessor, der zu Folgendem konfiguriert ist:
Bestimmen, durch Analysieren des ersten physiologischen Parameters, dass das Subjekt während eines ersten Zeitintervalls eine Behandlung erhält;
als Reaktion auf das Bestimmen, dass das Subjekt die Behandlung während des ersten Zeitintervalls erhält, Bestimmen, durch Analysieren des zweiten physiologischen Parameters, der während eines von dem ersten Zeitintervall unterschiedlichen zweiten Zeitintervalls erfasst wurde, eines Zustands des Subjekts; und
Veranlassen der Ausgabevorrichtung, eine Angabe über den Zustand auszugeben;
wobei der zweite physiologische Parameter eine Blutflussrate, ein Nettoflussvolumen oder eine volumetrische Flussrate einschließt,
wobei die medizinische Vorrichtung ferner einen dritten Sensor (308) einschließt, der dazu konfiguriert ist, einen Blutdruck des Subjekts zu erfassen, und
wobei der Prozessor dazu konfiguriert ist, den zweiten physiologischen Parameter und den Blutdruck des Subjekts zu analysieren, um den Zustand des Subjekts zu bestimmen.

2. Medizinische Vorrichtung nach Anspruch 1, wobei die Behandlung eine Brustkompression, einen Schrittmacherimpuls, eine assistierte Beatmung oder einen Elektroschock umfasst und
wobei der Zustand einen Puls, eine pulslose elektrische Aktivität (PEA), einen Anfall, eine Spontanatmung oder eine Arrhythmie umfasst.

3. Medizinische Vorrichtung nach einem der Ansprüche 1 bis 2, wobei der Prozessor dazu konfiguriert ist,
durch Analysieren des zweiten physiologischen Parameters, der während des von dem ersten Zeitintervall unterschiedlichen zweiten Zeitintervalls erfasst wurde, den Zustand des Subjekts zu bestimmen, durch:
Identifizieren, in dem zweiten physiologischen Parameter, eines Artefakts, das indikativ für den Zustand ist.

4. Medizinische Vorrichtung nach einem der Ansprüche 1 bis 3, wobei die Ausgabevorrichtung dazu konfiguriert ist, die Angabe des Zustands durch Übertragen, an eine mechanische Brustkompressionsvorrichtung, einer Anweisung auszugeben, das Anwenden von Brustkompressionen bei dem Subjekt zu beenden.

5. Medizinische Vorrichtung nach einem der Ansprüche 1 bis 4, wobei die Ausgabevorrichtung dazu konfiguriert ist, eine Wellenform anzuzeigen, die indikativ für den zweiten physiologischen Parameter ist, wobei die Wellenform einen ersten Abschnitt umfasst, der dem ersten Zeitintervall entspricht und einen zweiten Abschnitt, der dem zweiten Zeitintervall entspricht,
wobei der erste Abschnitt eine andere Farbe oder einen anderen Stil umfasst als der zweite Abschnitt.

6. Medizinische Vorrichtung nach einem der Ansprüche 1 bis 5, wobei:
der erste Sensor einen Beschleunigungsmesser umfasst, der dazu konfiguriert ist, eine Beschleunigung eines Brustkorbs eines Subjekts über das erste Zeitintervall und das zweite Zeitintervall zu erfassen;
der zweite Sensor einen Pulssensor umfasst, der dazu konfiguriert ist, einen Parameter zu erfassen, der indikativ für den Blutfluss durch ein Blutgefäß des Subjekts über das erste Zeitintervall und das zweite Zeitintervall ist;
der Prozessor konfiguriert ist zum:
Erfassen, durch Analysieren der Beschleunigung des Brustkorbs des Subjekts, einer ersten Brustkompression, die an dem Subjekt während des ersten Zeitintervalls durchgeführt wird;
Erfassen, durch Analysieren der Beschleunigung des Brustkorbs des Subjekts, des Fehlens einer zweiten Brustkorbkompression, die an dem Subjekt während des zweiten Zeitintervalls durchgeführt wird;
als Reaktion auf das Erfassen des Fehlens der zweiten Brustkorbkompression
die an dem Subjekt während des zweiten Zeitintervalls durchgeführt wird:
Erfassen, durch Analysieren des Parameters, der indikativ für den Blutfluss durch das Blutgefäß des Subjekts ist, eines Pulses des Subjekts während des zweiten Zeitintervalls; und
Veranlassen der Ausgabevorrichtung, ein Signal auszugeben, das den Puls angibt.

7. Medizinische Vorrichtung nach Anspruch 6, wobei der Prozessor dazu konfiguriert ist, den Puls des Subjekts während des zweiten Zeitintervalls zu erfassen, durch:
Berechnen eines Derivats des Blutflusses während des zweiten Zeitintervalls in Bezug auf die Zeit; und
Bestimmen, dass das Derivat abnimmt und den Nullpunkt durchquert.

8. Medizinische Vorrichtung nach Anspruch 6 oder 7, wobei der Pulssensor Folgendes umfasst:
einen Sender, der dazu konfiguriert ist, einen einfallenden Strahl auszugeben; und
einen Empfänger, der dazu konfiguriert ist, den Parameter zu erfassen, der indikativ für den Blutfluss durch das Blutgefäß des Subjekts ist, durch Erfassen einer Reflexion oder Streuung des einfallenden Strahls von dem Blutfluss durch das Blutgefäß des Subjekts, und
wobei der einfallende Strahl Ultraschall oder Infrarotlicht umfasst.

9. Verfahren, umfassend:
Identifizieren eines ersten physiologischen Parameters eines Subjekts, der von einem ersten Sensor erfasst wird;
Identifizieren eines zweiten physiologischen Parameters des Subjekts, der von einem zweiten Sensor erfasst wird,
wobei der zweite physiologische Parameter eine Blutflussrate, ein Nettoflussvolumen oder eine volumetrische Flussrate einschließt;
Erfassen, durch einen dritten Sensor, eines Blutdrucks des Subjekts;
Bestimmen, durch Analysieren des ersten physiologischen Parameters, dass das Subjekt während eines ersten Zeitintervalls eine Behandlung erhält;
als Reaktion auf das Bestimmen, dass das Subjekt die Behandlung während des ersten Zeitintervalls erhält, Bestimmen, durch Analysieren des zweiten physiologischen Parameters, der während eines von dem ersten Zeitintervall unterschiedlichen zweiten Zeitintervalls erfasst wurde, eines Zustands des Subjekts; und
Ausgeben einer Angabe über den Zustand,
wobei der Zustand des Subjekts durch Analysieren des zweiten physiologischen Parameters und des Blutdrucks des Subjekts bestimmt wird.

10. Verfahren nach Anspruch 9, wobei der zweite physiologische Parameter ein Volumen eines Blutgefäßes des Subjekts umfasst,
wobei das Bestimmen des zweiten physiologischen Parameters des Subjekts, der von einem zweiten Sensor erfasst wird, das Bestimmen des zweiten physiologischen Parameters während einer ersten Instanz eines zyklischen Ereignisses und während einer zweiten Instanz des zyklischen Ereignisses umfasst,
wobei das Verfahren ferner Folgendes umfasst:
Erfassen eines Blutdrucks des Blutgefäßes des Subjekts während der ersten Instanz eines zyklischen Ereignisses und während der zweiten Instanz des zyklischen Ereignisses, wobei die erste Instanz und die zweite Instanz während des zweiten Zeitintervalls auftreten;
Bestimmen einer Neigung einer ersten Druck-Volumen-Schleife (PV-Schleife), die dem Blutdruck und dem zweiten physiologischen Parameter während der ersten Instanz des zyklischen Ereignisses entspricht;
Bestimmen einer Neigung einer zweiten PV-Schleife, die dem Blutdruck und dem zweiten physiologischen Parameter während der zweiten Instanz des zyklischen Ereignisses entspricht; und
Bestimmen einer Differenz zwischen der Neigung der ersten PV-Schleife und der Neigung der zweiten PV-Schleife,
wobei das Bestimmen des Zustands des Subjekts das Bestimmen umfasst, dass die Differenz zwischen der Neigung der ersten PV-Schleife und der Neigung der zweiten PV-Schleife kleiner als ein erster Schwellenwert oder größer als ein zweiter Schwellenwert ist, und
wobei das zyklische Ereignis einen Herzzyklus, einen Brustkompressionszyklus oder einen kardiopulmonalen Wiederbelebungszyklus (CPR-Zyklus) umfasst.

11. Verfahren nach einem der Ansprüche 9 oder 10, wobei die Behandlung eine Brustkompression, einen Schrittmacherimpuls, eine assistierte Beatmung oder einen Elektroschock umfasst und wobei der Zustand einen Puls, eine pulslose elektrische Aktivität (PEA), einen Anfall, eine Spontanatmung, die Verabreichung eines Medikaments oder eine Arrhythmie umfasst; und/oder
wobei das Bestimmen, durch Analysieren des zweiten physiologischen Parameters, der während des von dem ersten Zeitintervall unterschiedlichen zweiten Zeitintervalls erfasst wurde, des Zustands des Subjekts das Erfassen, in dem zweiten physiologischen Parameter, eines Artefakts umfasst, das indikativ für den Zustand ist.

12. Verfahren nach einem der Ansprüche 9 bis 11, wobei
das Ausgeben der Angabe des Zustands das Übertragen, an eine mechanische Brustkompressionsvorrichtung, einer Anweisung umfasst, das Anwenden von Brustkompressionen bei dem Subjekt zu beenden; und/oder
das Ausgeben der Angabe des Zustands das Ausgeben, an einen Benutzer, einer Anweisung umfasst, dem Subjekt eine zweite Behandlung zu verabreichen; und/oder
wobei das Ausgeben der Angabe des Zustands Folgendes umfasst: Ausgeben der Angabe, dass der Zustand stabil ist, sich verschlimmert oder verbessert.

13. Verfahren nach einem der Ansprüche 9 bis 12, ferner umfassend:
Anzeigen einer Wellenform, die indikativ für den zweiten physiologischen Parameter ist, wobei die Wellenform einen ersten Abschnitt, der dem ersten Zeitintervall entspricht, und einen zweiten Abschnitt, der dem zweiten Zeitintervall entspricht, umfasst,
wobei der erste Abschnitt eine andere Farbe oder einen anderen Stil aufweist als der zweite Abschnitt.

## Revendications

1. Dispositif médical, comprenant :
un premier capteur (106) configuré pour détecter un premier paramètre physiologique d'un sujet (104) ;
un deuxième capteur (108, 304) configuré pour détecter un deuxième paramètre physiologique du sujet ;
un dispositif de sortie (114) ; et
un processeur configuré pour :
déterminer, en analysant le premier paramètre physiologique, que le sujet est en train de recevoir un traitement pendant un premier intervalle de temps ;
en réponse au fait de déterminer que le sujet est en train de recevoir le traitement pendant le premier intervalle de temps, déterminer, en analysant le deuxième paramètre physiologique détecté pendant un deuxième intervalle de temps qui est différent du premier intervalle de temps, un état du sujet ; et
amener le dispositif de sortie à sortir une indication de l'état ;
dans lequel le deuxième paramètre physiologique inclut un débit de flux sanguin, un volume de flux net ou un débit de flux volumétrique,
dans lequel le dispositif médical inclut en outre un troisième capteur (308) configuré pour détecter une pression artérielle du sujet, et
dans lequel le processeur est configuré pour analyser le deuxième paramètre physiologique et la pression artérielle du sujet afin de déterminer l'état du sujet.

2. Dispositif médical selon la revendication 1, dans lequel le traitement comprend une compression thoracique, un pouls de stimulation, une ventilation assistée ou un choc électrique, et
dans lequel l'état comprend un pouls, une activité électrique sans pouls (*pulseless electrical activity,* PEA), une attaque, une ventilation spontanée ou une arythmie.

3. Dispositif médical selon l'une quelconque des revendications 1-2, dans lequel le processeur est configuré pour déterminer, en analysant le deuxième paramètre physiologique détecté pendant le deuxième intervalle de temps qui est différent du premier intervalle de temps, l'état du sujet en :
identifiant, dans le deuxième paramètre physiologique, un artefact indiquant l'état.

4. Dispositif médical selon l'une quelconque des revendications 1-3, dans lequel le dispositif de sortie est configuré pour sortir l'indication de l'état en transmettant, vers un dispositif de compression thoracique mécanique, une instruction disant de cesser d'appliquer des compressions thoraciques au sujet.

5. Dispositif médical selon l'une quelconque des revendications 1-4, dans lequel le dispositif de sortie est configuré pour afficher une forme d'onde indiquant le deuxième paramètre physiologique, la forme d'onde comprenant une première partie correspondant au premier intervalle de temps et une deuxième partie correspondant au deuxième intervalle de temps,
dans lequel la première partie comprend une couleur ou un style différents de ceux de la deuxième partie.

6. Dispositif médical selon l'une quelconque des revendications 1-5, dans lequel :
le premier capteur comprend un accéléromètre configuré pour détecter une accélération d'un thorax d'un sujet pendant le premier intervalle de temps et le deuxième intervalle de temps ;
le deuxième capteur comprend un capteur de pouls configuré pour détecter un paramètre indiquant un flux sanguin à travers un vaisseau sanguin du sujet pendant le premier intervalle de temps et le deuxième intervalle de temps ;
le processeur est configuré pour :
détecter, en analysant l'accélération du thorax du sujet, une première compression thoracique réalisée sur le sujet pendant le premier intervalle de temps ;
détecter, en analysant l'accélération du thorax du sujet, une absence d'une deuxième compression thoracique réalisée sur le sujet pendant le deuxième intervalle de temps ;
en réponse au fait de détecter l'absence de la deuxième compression thoracique réalisée sur le sujet pendant le deuxième intervalle de temps :
détecter, en analysant le paramètre indiquant un flux sanguin à travers le vaisseau sanguin du sujet, un pouls du sujet pendant le deuxième intervalle de temps ; et
amener le dispositif de sortie à sortir un signal indiquant le pouls.

7. Dispositif médical selon la revendication 6, dans lequel le processeur est configuré pour détecter le pouls du sujet pendant le deuxième intervalle de temps en :
calculant une dérivée du flux sanguin pendant le deuxième intervalle de temps par rapport au temps ; et
déterminant que la dérivée décroît et passe en dessous de zéro.

8. Dispositif médical selon la revendication 6 ou 7, dans lequel le capteur de pouls comprend :
un émetteur configuré pour sortir un faisceau incident ; et
un récepteur configuré pour détecter le paramètre indiquant un flux sanguin à travers le vaisseau sanguin du sujet en détectant une réflexion ou une diffusion du faisceau incident depuis le flux sanguin à travers le vaisseau sanguin du sujet, et
dans lequel le faisceau incident comprend une lumière à ultrasons ou infrarouge.

9. Procédé, comprenant :
l'identification d'un premier paramètre physiologique d'un sujet détecté par un premier capteur ;
l'identification d'un deuxième paramètre physiologique du sujet détecté par un deuxième capteur ;
la détection, par un troisième capteur, d'une pression artérielle du sujet ;
le fait de déterminer, en analysant le premier paramètre physiologique, que le sujet est en train de recevoir un traitement pendant un premier intervalle de temps ;
en réponse au fait de déterminer que le sujet est en train de recevoir le traitement pendant le premier intervalle de temps, le fait de déterminer, en analysant le deuxième paramètre physiologique détecté pendant un deuxième intervalle de temps qui est différent du premier intervalle de temps, un état du sujet ; et
la sortie d'une indication de l'état,
dans lequel l'état du sujet est déterminé en analysant le deuxième paramètre physiologique et la pression artérielle du sujet.

10. Procédé selon la revendication 9, dans lequel le deuxième paramètre physiologique comprend un volume d'un vaisseau sanguin du sujet,
dans lequel le fait de déterminer le deuxième paramètre physiologique du sujet détecté par un deuxième capteur comprend le fait de déterminer le deuxième paramètre physiologique pendant une première instance d'un événement cyclique et pendant une deuxième instance de l'événement cyclique,
dans lequel le procédé comprend en outre :
la détection d'une pression artérielle du vaisseau sanguin du sujet pendant la première instance d'un événement cyclique et pendant la deuxième instance de l'événement cyclique, la première instance et la deuxième instance survenant pendant le deuxième intervalle de temps ;
la détermination d'une pente d'une première boucle pression-volume (PV) correspondant à la pression artérielle et au deuxième paramètre physiologique pendant la première instance de l'événement cyclique ;
la détermination d'une pente d'une deuxième boucle PV correspondant à la pression artérielle et au deuxième paramètre physiologique pendant la deuxième instance de l'événement cyclique ; et
la détermination d'une différence entre la pente de la première boucle PV et la pente de la deuxième boucle PV,
dans lequel la détermination de l'état du sujet comprend le fait de déterminer que la différence entre la pente de la première boucle PV et la pente de la deuxième boucle PV est inférieure à un premier seuil ou supérieure à un deuxième seuil, et
dans lequel l'événement cyclique comprend un cycle cardiaque, un cycle de compression thoracique ou un cycle de réanimation cardiopulmonaire (*cardiopulmonary resuscitation,* CPR).

11. Procédé selon l'une quelconque des revendications 9 ou 10, dans lequel le traitement comprend une compression thoracique, un pouls de stimulation, une ventilation assistée ou un choc électrique, et dans lequel l'état comprend un pouls, une activité électrique sans pouls (PEA), une attaque, une ventilation spontanée, une administration de médicaments ou une arythmie ; et/ou
dans lequel le fait de déterminer, en analysant le deuxième paramètre physiologique détecté pendant le deuxième intervalle de temps qui est différent du premier intervalle de temps, l'état du sujet comprend la détection, dans le deuxième paramètre physiologique, d'un artefact indiquant l'état.

12. Procédé selon l'une quelconque des revendications 9-11, dans lequel
la sortie de l'indication de l'état comprend la transmission, vers un dispositif de compression thoracique mécanique, d'une instruction disant de cesser d'appliquer des compressions thoraciques au sujet ; et/ou
la sortie de l'indication de l'état comprend la sortie, vers un utilisateur, d'une instruction disant d'administrer un deuxième traitement au sujet ; et/ou
dans lequel la sortie de l'indication de l'état comprend :
la sortie de l'indication disant que l'état est stable, s'aggrave ou s'améliore.

13. Procédé selon l'une quelconque des revendications 9-12, comprenant en outre :
l'affichage d'une forme d'onde indiquant le deuxième paramètre physiologique, la forme d'onde comprenant une première partie correspondant au premier intervalle de temps et une deuxième partie correspondant au deuxième intervalle de temps,
dans lequel la première partie comprend une couleur ou un style différents de ceux de la deuxième partie.
